# EUROPEAN PATENT SPECIFICATION

(11) **EP 1 119 528 B1**
(45) Date of publication and mention of the grant of the patent: **28.08.2002**
(21) Application number: 99947748.2
(22) Date of filing: 05.10.1999
(51) Int. Cl.: C07B 61/00, C07D 239/38

(54) **CHEMICAL CONSTRUCTS AND THEIR USES**
CHEMISCHE KONSTRUKTIONEN UND DEREN VERWENDUNGEN
PRODUITS DE SYNTHESE CHIMIQUES ET LEUR UTILISATION

(30) Priority: 05.10.1998 GB 9821669
(43) Date of publication of application: 01.08.2001
(73) Proprietor: GLAXO GROUP LIMITED, Greenford, Middlesex UB6 ONN (GB)
(72) Inventor: McKEOWN, Stephen Carl Glaxo Wellcome plc, Stevenage Hertfordshire SG1 2NY (GB); WATSON, Stephen Paul Glaxo Wellcome plc, Stevenage Hertfordshire SG1 2NY (GB)
(74) Representative: Stott, Michael John
(86) International application number: GB9903284
(87) International publication number: WO00020112

(56) References cited:
- WO-A-95/28640
- WO-A-97/37953
- CARRASCO M R ET AL: "Direct Monitoring of Organic Reactions on Polymeric Supports" TETRAHEDRON LETTERS,NL,ELSEVIER SCIENCE PUBLISHERS, AMSTERDAM, vol. 38, no. 36, 8 September 1997 (1997-09-08), pages 6331-6334, XP004087929 ISSN: 0040-4039
- GEYSEN H M ET AL: "ISOTOPE OR MASS ENCODING OF COMBINATORIAL LIBRARIES" CHEMISTRY AND BIOLOGY,GB,CURRENT BIOLOGY, LONDON, vol. 3, no. 8, 1 August 1996 (1996-08-01), pages 679-688, XP002035873 ISSN: 1074-5521

## Description

### Field of the Invention

This invention relates to chemical constructs for use in solid phase synthesis, and to methods of detecting and/or characterising the products of solid phase synthesis using the constructs.

### Background of the Invention

Solid phase synthesis has been known for many years in the field of peptide synthesis and more recently has also been used increasingly for the synthesis of non-peptides.

Solid phase synthesis has found particular application in the field of combinatorial chemistry and the preparation of chemical libraries as potential sources of new leads for drug discovery, see for example Anthony W. Czarnik, *Analytical Chemistry News and Features,* June 1, 1998, pp 378A-386A, and *The Combinatorial Index,* Barry A. Bunin, Academic Press, San Diego 1998. A feature of combinatorial chemistry methods is that they enable very large numbers of different compounds to be prepared from a relatively limited number of molecular building blocks in a relatively small number of reactions. Combinatorial chemistry makes use of the "split and pool" approach in which a suspension of chemical starting material tethered to a solid support is split into N portions, each of which is reacted with a different reagent. The products of the N reactions are then pooled and mixed thoroughly, the resulting pool is split into N' portions and again each portion is reacted with a different reagent. This procedure can be repeated as many times as there are steps in the reaction sequence. Thus, for a three step reaction sequence, if the reaction mixture is divided into ten portions at each stage, each of the pools being reacted with a different reagent before recombining with the other portions, the total number of compounds formed by the process will be 10³ = 1000. Thus it can be seen that by using the split and pool technique, a large number of different molecules can be synthesised using a minimal number of reactions (thirty in the case mentioned above).

Each of the solid supports (e.g. a resin bead) will have a single product molecule tethered to it and hence in principle each of the reaction products can be separated and analysed or subjected to biological testing simply by isolating each single solid support and cleaving the product from the support. However, the numbers of compounds generated in large libraries by combinatorial methods means that it can be impracticable to identify and characterise each compound. Consequently, the compounds usually are first tested, either on the solid support or after cleaving from the support, and only those compounds which show some biological activity are subsequently identified. In order to minimise the number of biological tests carried out, compounds can be tested in pools containing a predetermined number of compounds, the inactive pools being discarded and the active pools being subject to further investigation. The biological activities of the compounds can be analysed using high throughput automated assay techniques permitting large numbers of compounds to be analysed in a short time.

One of the problems facing the chemist is how to identify and characterise the various compounds in a combinatorial library since each compound will be present in the library only at very low concentrations, and there will usually be insufficient compound present on a given solid support to allow for both biological testing and identification of the compound. This problem has been addressed by providing each solid support with a coding tag from which the identity of the compound can be determined. Thus, for example, a coding tag can be built up in sequential steps on the solid support in parallel with the construction of the desired target compound, the coding tag reflecting the synthesis history of the product compound and being unique for each product compound. The coding tag is usually built up on the support using chemical reactions of a type which are orthogonal to the chemistry used to build up the product compound, thereby ensuring that the coding units and product compounds do not become confused. Once the product compound has been tested, and its biological activity confirmed, the coding tag can then be decoded to allow identification of the compound.

Where a product compound has been subjected to a preliminary biological screen as. part of a pool of compounds, and the pool has been shown to have activity, it is then necessary either to look at smaller pools of compounds or individual compounds in order to discover the identity of the active compound. In many assay systems, it is necessary to cleave the compound from the solid support in order to carry out biological testing, and this can lead to problems with the pool testing approach. Thus, if all of the active compound has been cleaved from the solid support in order to carry out the testing, such that the pool contains a mixture of a large number of dissolved compounds, recovery of each individual compound for further testing would create a severe separation problem. In order to overcome or avoid this problem, "differential release" constructs have been created in which the product compound, e.g. a drug molecule, is linked to the solid support by several different types of linker groups, each of the linker groups being orthogonally and selectively cleavable under different chemical conditions. Such a construct (Construct A) is shown schematically below.

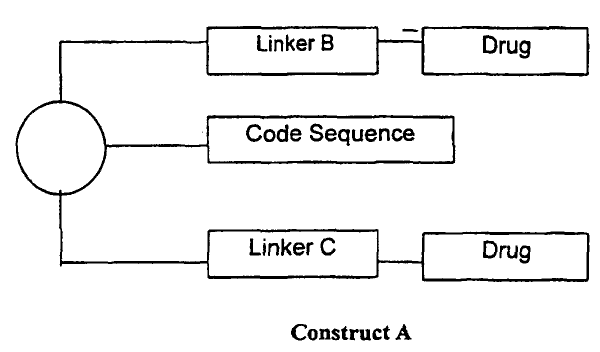

Following a combinatorial synthesis scheme, the reaction products can be divided into x pools each containing y solid supports, each solid support bearing a different product drug molecule. Each pool can then be subjected to cleavage conditions suitable for selectively cleaving linker B so as to free a proportion of the total drug present on the solid support. The solid supports can then be removed and the residual solution subjected to the desired biological assays. If the pool as a whole shows biological activity, the individual partially cleaved solid supports in that active pool can then be separated into individual containers and subjected to conditions effective to bring about cleavage of the second linker, linker C. Subsequently, the individual drugs can each be screened for biological activity.

Once biological activity has been observed for drug associated with a particular individual solid support, the support is subjected to conditions which allow the tagging code to be decoded and the identity of the drug compound established.

An alternative to "differential release" is "tiered release". In a tiered release system, the construct does not employ two orthogonal cleavage sites as with the differential release constructs, but makes use of only a single cleavage site. A proportion of the drug or test compound is released in a first cleavage step to provide sufficient compound for a first biological assay, and subsequently more drug can be released by cleavage at the same cleavage site for further biological assays. Such tiered release systems make use of cleavage reactions which are relatively slow, and where the rate of cleavage can be reasonably well controlled. Photochemical cleavage is typically used in tiered release systems. A construct for use in a tiered release system is shown below as Construct B.

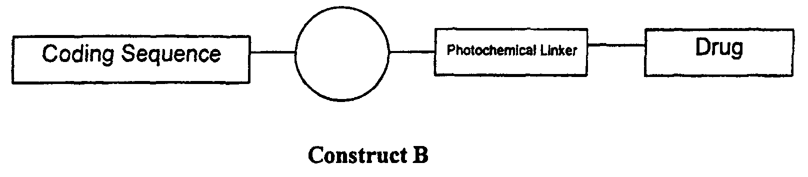

In any solid phase synthesis procedure, whether or not part of a combinatorial procedure, it is important to be able to determine the optimal conditions for a given reaction step in a series of steps. It is also important to be able to monitor the progress or path of a reaction so that it can be determined whether a particular reaction has gone to completion, or indeed whether a particular reaction has taken place at all. This is particularly important in a multistage solid phase synthesis where the failure of a given stage to proceed to completion can lead to the formation of side products thereby complicating what is otherwise a relatively straightforward separation procedure.

Although both invasive and non-invasive analytical methods are known for determining the products of solid phase synthesis (see *The Combinatorial Index*, *idem*), one of the recognised problems with solid phase synthesis is that it is generally more difficult to monitor the progress of a reaction; see for example the Czarnik paper referred to above. This is particularly true with the products of combinatorial chemistry methods where there is a need to use rapid high throughput techniques to analyse the large numbers of compounds generated by such methods. Techniques such as mass spectrometry are potentially ideally suited as a means of providing high throughput analyses, but a substantial problem is that not all compounds produce a guaranteed response under mass spectrometric conditions. Indeed, many compounds are "invisible" to the so-called "soft" methods of mass spectrometry such as Matrix-Assisted Laser-Desorption Ionisation (MALDI) and Electrospray mass spectrometry which are intended to detect molecular ions without causing significant fragmentation of the molecule. One of the reasons for this is that under MALDI and electrospray conditions, many compounds, particularly peptides, do not ionise sufficiently well to give a detectable mass spectral response.

It is an object of the present invention to provide a means of monitoring the progress or path of a chemical reaction on a solid support, for example in a combinatorial synthesis, which avoids problems inherent in known methods and which provides a means of detecting and identifying the products of solid phase synthetic techniques using mass spectroscopy.

Accordingly, in a first aspect, the invention provides a chemical construct for use in solid phase synthesis comprising a solid support Q having linked thereto groups Y¹R and Y²R; wherein R is a substrate or a coding tag and the groups Y¹ and Y² are connecting groups each having a first cleavage site, at least one of Y¹ and Y² having a second cleavage site located between the first cleavage site and group R, the first cleavage site being orthogonally and selectively cleavable with respect to the second cleavage site, and, when both groups Y¹ and Y² contain a second cleavage site, the second cleavage site in Y¹ being selectively and orthogonally cleavable with respect to the second cleavage site in Y²; the second cleavage site being cleavable to release the substrate; and the first cleavage site being selectively cleavable to release a fragment Fr comprising the substrate R and at least a portion of the connecting group Y; and wherein:
(i) the chemical fragment Fr contains a sensitising group G which sensitises the chemical fragment Fr to instrumental, e.g. mass spectroscopic analysis and/or:
(ii) the fragment Fr contains a means for imparting a characteristic signature to the mass spectrum of the fragment.

Preferably at least one group R is a substrate and more preferably the substrate is a drug molecule or other molecule having biological activity.

In one preferred embodiment, there is provided a chemical construct for use in solid phase synthesis comprising a solid support Q having linked thereto groups Y¹R and Y²R; wherein R is a substrate (such as a drug molecule) and the groups Y¹ and Y² are connecting groups each having first and second cleavage sites which are orthogonally and selectively cleavable, the second cleavage site in Y¹ being selectively and orthogonally cleavable with respect to the second cleavage site in Y²; the second cleavage site being cleavable to release the substrate; and the first cleavage site being located at a position between the second cleavage site and the solid support and being selectively cleavable to release a fragment Fr comprising the substrate R and at least a portion of the connecting group Y; and wherein:
(i) the chemical fragment Fr contains a sensitising group G which sensitises the chemical fragment Fr to instrumental, e.g. mass spectroscopic analysis and/or:
(ii) the fragment Fr contains a means for imparting a characteristic signature to the mass spectrum of the fragment.

The constructs of the invention can be illustrated by the general formula:

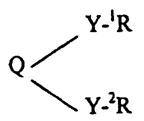

The constructs of the invention provide a differential release system which is more readily analysable to determine the outcome of individual reactions on the solid support. Thus, for example, by subjecting the construct to cleavage conditions effective selectively to bring about cleavage at each of the first cleavage sites, chemical fragments containing the substrates R linked to an "analytical lever" are produced in which the detectability of the chemical fragments, and hence the substrates, is enhanced. Thus, cleavage at the first cleavage sites can be used to determine whether a particular substrate has been formed, a facility which is useful as a means of carrying out quality control (QC) during a reaction scheme or library generation, and which provides a means of analysis that can be used when optimising the conditions for a given synthetic process step.

The constructs can contain further groups Yⁿ, each of which has a second cleavage site which is selectively and orthogonally cleavable with respect to the second cleavage sites in the other Y groups. Thus, for example, the constructs can contain a group Y³Q in which the second cleavage site in the group Y³ is cleaved under different chemical conditions from the second cleavage sites in Y¹R and Y²R. Thus by means of the invention, it is possible to provide differential release of product substrate molecules (e.g. drugs) in two, three, four or more steps, thereby assisting the products of large combinatorial libraries to be screened biologically in pools of manageable size.

In a second aspect, the invention provides a differential release method of assaying a chemical library for biological activity, the method comprising:
(i) subjecting a construct comprising a solid support Q having linked thereto groups Y¹R and Y²R as hereinbefore defined to cleavage conditions effective to release substrate R from the group Y¹R;
(ii) testing the substrate R released from the group Y¹R in a biological assay;
(iii) subsequently subjecting the construct to cleavage conditions effective to release substrate R from the group Y²R; and
(iv)) testing the substrate R released from the group Y²R in a biological assay.

In addition to providing a differential release method of analysing a library, the invention also provides a method of tiered release analysis which makes use of constructs which are selectively cleavable to release a fragment Fr comprising the substrate R and at least a portion of a connecting group Y; and wherein:
(i) the chemical fragment Fr contains a sensitising group G which sensitises the chemical fragment Fr to instrumental, e.g. mass spectroscopic analysis and/or:
(ii) the fragment Fr contains a means for imparting a characteristic signature to the mass spectrum of the fragment.

Accordingly, in a further aspect, the invention provides a tiered release method of assaying a chemical library for biological activity, the method comprising:
(i) subjecting a construct comprising a solid support Q having linked thereto a group Y¹R as hereinbefore defined to cleavage conditions effective to release a first portion of the substrate R from the group Y¹R;
(ii) testing the first portion of substrate R released from the group Y¹R in a biological assay;
(iii) subjecting the construct to cleavage conditions effective to release a second portion of the substrate R from the group Y¹R; and
(iv) testing the second portion of substrate R released from the group Y¹R in a biological assay.

In each of the above differential release and tiered release methods of the invention, the cleavage step (i) is typically performed on a pool of solid supports, each bearing a different substrate R and, following the cleavage step (I), the solid support is typically separated from the released substrate R. If biological activity is detected in the pool, the solid supports can be isolated, or grouped into smaller pools, and then subjected to cleavage step (iii) to release further substrate for testing so that the biological activity can be traced to a particular solid support or small group of supports.

To assist in determining the identities of the products of a combinatorial synthesis scheme, each solid support can contain a coding tag or coding sequence which encodes information indicative of at least part of the synthesis history of the construct. The coding tag can be built into the solid support itself or a coding sequence can be linked to the solid support. The coding sequence preferably is linked to the solid support by means of a connecting group Y^{a} having a cleavage site cleavable to release a fragment F^{a} from the solid support, the fragment F^{a} comprising the coding sequence and optionally at least a portion of the connecting group Y^{a}.

It is preferred that:
(i) the chemical fragment F^{a} contains a sensitising group G which sensitises the chemical fragment F^{a} to instrumental, e.g. mass spectroscopic analysis and/or:
(ii) the fragment F^{a} contains a means for imparting a characteristic signature to the mass spectrum of the fragment.

The coding tag can take the form of a group or sequence of coding groups that can be fragmented, and in particularly sequentially fragmented, under mass spectrometric conditions to provide one or more characteristic mass spectral peaks that provide a code from which the substrate can be identified. For example, the coding tag can comprise a sequence of different amino acids, e.g. two, three or four amino acids, the identity and order of the amino acids forming a code indicative of the identity of the substrate. Under mass spectrometry conditions, the coding groups (e.g. amino acids) can be sequentially cleaved to give a series of characteristic mass spectral fragments, relating to coding tags in which ,for example, one or two or more amino acids have been lost from the tag. By relating the masses of the fragment peaks back to the mass of the parent coding tag, it is possible to identify the individual coding groups (e.g. amino acids) and their position on the coding tag, thereby enabling the code to be broken and the substrate identified. By providing a sensitising group G and a means for imparting a characteristic signature to the mass spectrum, the present invention provides a means firstly of ensuring that the amino acids or other coding groups are sufficiently ionised to give strong mass spectral peaks, and secondly of ensuring that each of the characteristic mass spectral fragments formed from the fragmentation of the coding sequence can be distinguished from extraneous peaks.

Accordingly, in another aspect, the invention provides a method of determining the identity of a substrate R linked to a solid support Q by mass spectrometric means; the solid support Q having a coding sequence attached thereto by means of a connecting group Y^{a} having a cleavage site cleavable to release a fragment F^{a} from the solid support, the fragment F^{a} comprising the coding sequence and at least a portion of the connecting group Y^{a}, wherein
(i) the chemical fragment F^{a} contains a sensitising group G which sensitises the chemical fragment F^{a} to mass spectroscopic analysis; and preferably
(ii) the fragment F^{a} contains a means for imparting a characteristic signature to the mass spectrum of the fragment;
the coding sequence comprising a sequence of coding groups the nature and order of which is indicative of the identity of the substrate R;
the method comprising cleaving the connecting group Y^{a} so as to release the fragment F^{a} from the solid support; subjecting the fragment Y^{a} to mass spectrometry under conditions effective to bring about mass spectral fragmentation of the coding group and the formation of mass spectral, fragment ions corresponding to the loss of one or more coding groups from the coding sequence, and thereafter correlating mass spectral peaks of the mass spectral fragment ions with the molecular ion of the fragment Y^{a} to identify the sequence of the individual coding groups

In the methods and constructs of the invention, as an alternative, or in addition, to including a coding tag which is chemically different from the substrate, a proportion of the substrate itself can be bonded to the solid support in such a way that it is not cleaved from the support under the conditions used to remove the rest of the substrate from the support. The remaining substrate can thus function as an analytical label enabling identification by a method such as mass spectrometry. More particularly, a proportion of the total substrate R in the construct can be linked to the solid support by means of a connecting group Y^{b} having a cleavage site which is cleavable to release a fragment F^{b} from the solid support, the fragment F^{b} comprising the substrate and at least a portion of the connecting group Y^{b}; the connecting group Y^{b} not being cleavable to release substrate R under conditions effective to cleave the second cleavage sites in the groups Y¹R, Y²R and Yⁿ [if present]) and wherein:
(i) the chemical fragment F^{b} contains a sensitising group G which sensitises the chemical fragment F^{a} to instrumental, e.g. mass spectroscopic analysis and/or:
(ii) the fragment F^{b} contains a means for imparting a characteristic signature to the mass spectrum of the fragment.

Thus, after liberation of the main proportion of the substrate from the constructs for biological testing, fragment F^{b} can be selectively cleaved from the solid support to yield sensitised substrate which is readily analysable by methods such as mass spectrometry. This could be done, for example, after biological assays had been carried out on the main proportion of the substrate, biological activity having been found, and it was required to determine the identity of the substrate. Alternatively, fragment F^{b} can be cleaved from the solid support without first removing the other substrate from the solid support, for example as part of a quality control (QC) procedure to test the quality of the products of the solid phase synthesis. In this latter procedure, fragment F^{b} can be cleaved from the solid support along with fragments Fr and F^{a}, analysis of the fragments Fr, F^{a} and F^{b} providing a full picture of the outcome of the synthesis.

The cleavage site of groups Y^{a} and Y^{b} is preferably cleavable under conditions corresponding to those needed to cleave the first cleavage sites in the groups Y¹R and Y²R. Thus, the product or substrate-containing fragments and the coding tag-containing fragment can be cleaved from the solid support at the same time thereby permitting them to be analysed (e.g. by mass spectrometry) simultaneously.

The chemical fragment F^{a} preferably contains a sensitising group G which sensitises the chemical fragment F^{a} to instrumental, e.g. mass spectroscopic analysis.

In a preferred aspect of the invention, the construct is characterised in that the sensitising group G is generated by cleavage at the first cleavage site(s) of the groups Y¹, Y², Yⁿ, Y^{a} or Y^{b} respectively. In this preferred embodiment of the invention, a moiety containing the sensitising group G is formed or introduced at the first cleavage site(s) by cleavage of the "skeleton" of the construct, as distinct from cleavage of a side chain or mere removal of a protecting group.

The sensitising group G renders the fragment Fr, F^{a} or F^{b}, and hence the substrate R, coding tag or substrate-containing coding tag, more sensitive to analysis by a given analytical technique, and in particular a mass spectrometric technique. Thus the sensitising group can be a group which is readily ionisable under the conditions encountered in a mass spectrometer, and in particular electrospray mass spectrometry, to afford a strong signal.

The ionisable sensitising group serves to ensure that the fragment Fr, F^{a} or F^{b} is ionised sufficiently in the mass spectrometer to give a strong response. This overcomes a problem inherent in many molecules synthesised by solid phase methods where a suitable ionising group is not present and analysis by high throughput mass spectral techniques is problematical.

The ionisable group can be for example a basic amino group or a carboxylate group but preferably it is a basic amino group. It will be appreciated that the term "basic amino group" as used herein refers in particular to an amino group which is readily protonated.

The basic amino group can be a primary amino group, a secondary amino group, or a tertiary amino group. Where the basic amino group is a tertiary amino group, it can be for example, a cyclic amino group such as piperidino, piperazino, pyrrolidino, or morpholino, piperidino or piperazino (e.g.N-methylpiperazino) being presently preferred.

In accordance with a preferred embodiment of the invention, a moiety containing the sensitising group G, such as a basic amino group, is formed or introduced at the cleavage site as the chemical fragment Fr, fragment F^{a} or F^{b} (hereinafter collectively referred to as the analytical fragment) is cleaved from the solid support. The advantage of forming the sensitising group G in this way is that it avoids the potential problem of a pre-existing or preformed sensitising group interfering with the chemistry of the construct.

The atoms or functional group making up the sensitising group G can be present in a masked form in the construct before cleavage of the analytical fragment from the resin, the cleavage conditions merely serving to unmask the sensitising group G. Alternatively, the atoms or functional group making up or containing the sensitising group G can be introduced at the cleavage site during the cleavage reaction. For example, where the sensitising group is a basic amino group, the nitrogen atom of the amino group can be present in the construct before cleavage, or it can be introduced during the cleavage reaction.

When the sensitising group G is introduced from an external source during the cleavage reaction, it can form part of a larger moiety. For example, the group G can be introduced as part of a group X-G wherein X is the residue of a nucleophile or electrophile, for example a nitrogen-based or sulphur-based nucleophile. Examples of groups X-G include groups of the formula G-Alk-Nuc wherein Alk is an alkylene group and Nuc is a nucleophile such as a secondary amino group (e.g. the secondary amino group in N-methylpiperazine) or a thiolate group.

It is preferred that the analytical fragment contains a means for imparting a characteristic signature to the mass spectrum of the fragment. The signature can advantageously be provided by incorporating into the fragment a "peak splitting" isotopic label. The peak splitting isotopic label comprises at least one atom that exists in a number of stable isotopic forms. By isotopic labelling of one or more particular atoms in the analytical fragment such that a given atom is labelled with a mixture of isotopes, the mass spectra of the molecular ions will appear as a characteristic pattern, the precise pattern depending on the relative amounts of the individual isotopes. Thus, for example, if a given atom in the fragment Fr is labelled such that 50% of the atoms are of one isotopic form and 50% are of another isotopic form, the mass spectrum will show the molecular ion as a characteristic doublet in which the peaks are of approximately equal height.

The purpose of the peak splitting atom(s) is to provide a characteristic pattern which will characterise any peak in the mass spectrum originating from the analytical fragment Fr and/or F^{a}, and/or F^{b} thereby distinguishing such peaks from those due to extraneous materials.

The analytical fragments Fr, F^{a} and F^{b} can be labelled the same or differently but in one preferred embodiment, they are labelled differently so as to produce different characteristic signatures. In this way, mass spectral peaks due to the coding tag can be distinguished from mass spectral peaks from the fragments containing the substrate.

Examples of atoms that can be used as isotopic peak splitting labels include ¹H/H² (D), ⁷⁹Br/⁸¹Br, ¹²C/¹³C, ¹⁴N/¹⁵N and ¹⁶O/¹⁸O.

The analytical fragments Fr, F^{b} and F^{a} can each contain a single peak splitting isotopic label or more than one such label. For example, the isotopic label can be a single bromine atom in which case the peak for the molecular ion of the analytical fragment liberated following cleavage from the solid support will appear as a doublet. By introducing a second or subsequent peak splitting label(s), a more complex peak pattern will be produced for the molecular ion.

The isotopic peak splitting label(s) preferably is/are located between the first and second cleavage sites of the groups Y¹ and Y².

The first and second cleavage sites in the groups Y¹ can be defined by first and second linker groups L¹ and L2, the first and second cleavage sites in the group Y² can be defined by first and second linker groups L¹ and L³, and the cleavage site in the group Y^{a} can be defined by a linker group L^{a}, which preferably corresponds to L^{1,} L¹, ,L² and L³ being selectively and orthogonally cleavable. A "spacer group" A can be interposed between each pair of first and second linker groups, or between the linker group L^{a} and the coding tag, the spacer group A typically containing an isotopic peak splitting label.

Accordingly, one preferred embodiment of the invention can be represented by the formula: 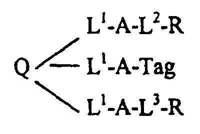 wherein "Tag" represents a coding sequence.

In another preferred embodiment, the construct has a substrate-containing analytical group R-A linked to the solid support by a linker L¹ which is orthogonally cleavable with respect to the linkers L² and L³. A construct of this type can be represented by the formula:

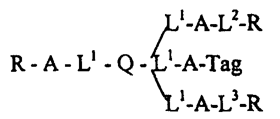

In a further preferred embodiment, the coding sequence is omitted and the means of identifying the substrate is provided by the group R - A - L¹ which can be cleaved to provide sensitised substrate for analysis by mass spectrometry. Such a construct has the formula:

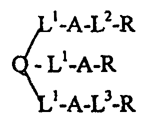

In another preferred embodiment, the invention provides a construct for use in a tiered release method of screening as described above, the construct containing a sensitised coding tag. Such a construct can be represented by the formula Tag - A - L¹ - Q - L¹ - A -L² -R.

In other embodiments of the invention, the constructs can have a formula selected from the group consisting of:

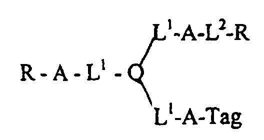

R-A-L¹-Q-L¹-A-L²-R

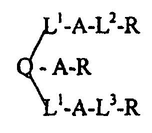

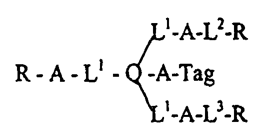

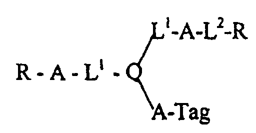

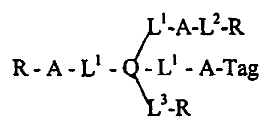

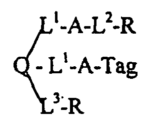

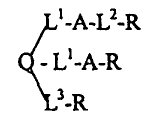

R-L³-Q-L¹-A-L²-R

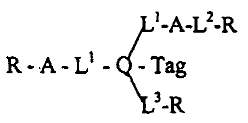

wherein L¹, L², L³, A and R are as hereinbefore defined and "Tag" represents a coding sequence.

The linkers L¹, L², and L³ are orthogonally and selectively cleavable; i.e. the conditions used to effect cleavage at the cleavage site in one linker will not cleave the other. A wide variety of different types of cleavage reaction can be used, examples being reactions selected from acid catalysed cleavage, base catalysed cleavage, oxidative cleavage, reductive cleavage, nucleophilic displacement, electrophilic displacement, and thermal, photochemical and enzymatic cleavage.

Thus, in a preferred embodiment, there is provided a chemical construct as hereinbefore defined wherein the first cleavage site (e.g. as defined by the linker L¹) is selectively cleavable by one type of chemistry selected from a group of chemistries consisting of cleavage under acid conditions, base catalysed cleavage, oxidative cleavage, reductive cleavage, nucleophilic displacement, electrophilic displacement, and thermal, photochemical and enzymatic cleavage, one of the second cleavage sites (e.g. as defined by the linker L²) is selectively cleavable by a second and different type of chemistry selected from the said group, and the other of the second cleavage sites (e.g. as defined by the linker L³) is selectively cleavable by a third and different type of chemistry selected from the said group.

Any one or more of the cleavage sites/linkers, can be of the "safety catch" variety; i.e. the cleavage site or linker group must be chemically modified in a first step before it can be subjected to cleavage in a second step. An advantage of such an arrangement is that it prevents or significantly reduces the possibility of cleavage taking place inadvertently. One example of a "safety catch" mechanism involves oxidation of a functional group in a first step, the oxidation serving to make the functional group more amenable to displacement by a nucleophile in a subsequent cleavage step. The nucleophile can vary considerably in structure. For example, in one embodiment, the nucleophile can be an amino group-containing nucleophile), the amino group participating in the nucleophilic displacement action such that the amino group is attached directly to the cleavage site. Alternatively, in another embodiment, the amino group (or other sensitising group) can be present in a group (e.g. a dialkylaminoalkyl-thiolate anion) containing another nucleophile such as a sulphur nucleophile which becomes attached to the cleavage site. In a further embodiment, the nucleophile can be a moiety containing a one basic nitrogen atom which functions as a nucleophile, and another basic nitrogen which functions as the sensitiser group G, an example of such a nucleophile being N-methylpiperazino.

Examples of linkers that can be selectively cleaved under acidic conditions include appropriately substituted benzyloxycarbonyl groups and substituted diphenyl-methylamino groups, both of which can be cleaved by the action of trifluoroacetic acid.

Particular examples of acid cleavable linker groups are set forth in *The Combinatorial Index,* Barry A. Bunin, Academic Press, San Diego 1998. Linkers of the "Rink" or "Knorr" type typically comprise an N-protected 1-amino-1,1-diphenylmethane moiety, the amino group when deprotected allowing attachment to a substrate, one of the phenyl rings being substituted for example with dimethoxy groups and the other having a carboxyalkyloxy substituent providing a second point of attachment. Cleavage with trifluoroacetic acid gives rise to a substrate compound having a terminal carboxamido group. Linkers of the "Wang" type typically contain a substituted phenoxyacetyl group, the acetyl group providing one point of attachment, and a benzylic hydroxyl group on the phenyl ring forming a second point of attachment. Esters can be formed between a carboxyl group of a substrate and the benzylic hydroxyl group, the ester groups being subsequently cleavable with trifluoroacetic acid (TFA) to release a substrate compound having a terminal carboxylate group.

Examples of groups that can be selectively cleaved under photochemical conditions include carbamate groups such as o-nitrobenzyloxycarbamate groups in which initial cleavage takes place between the benzyloxy and carbonyl groups, elimination of carbon dioxide from the resulting fragment giving a basic amino group.

Examples of groups that can be cleaved by nucleophilic displacement include mercaptopyrimidine-based "safety catch" linkers such as 5-carboxy-2-mercaptopyrimidine, where cleavage can be effected by reacting under oxidising conditions to generate a sulphoxide or sulphone linkage, followed by reaction with a nucleophilic amino group to form a 2-aminopyrimidine. Particular examples of nucleophilic groups that can be used to effect cleavage of oxidised mercaptopyrimidine-based linkers include cyclic amines such as piperidine and N-substituted piperazine (e.g. N-methylpiperazine), and amino group-containing thiolate nucleophiles (e.g. dimethylaminoethylthiolate). Examples of oxidising conditions are those generated oxidising agents such as per-acids, e.g. a perbenzoic acid such as *m*-chloroperbenzoic acid, and certain inorganic per-salts such as potassium monoperoxysulphate.

By making the first and second cleavage sites, e.g. as defined by the first and second linker groups L¹ and L², or L¹ and ³ orthogonally cleavable, it is possible selectively to separate from the construct either the analytical fragment or the substrate R, simply by using different cleavage conditions. This means that during experiments designed to optimise the conditions for a particular reaction step, the chemist can subject the construct to conditions suitable for cleaving off the analytical fragment thereby allowing analysis to be carried out to determine the outcome of each test reaction. Similarly, during a preparative scale reaction (e.g. a scale-up reaction or commercial production), quality control (QC) can be carried out by removing a number of solid supports from the reaction vessel, cleaving the constructs at the first cleavage site and analysing the resulting fragment to see whether a particular reaction step has worked. On the other hand, by cleaving at the second cleavage site e.g. on the second linker group, rather than the first, the reaction product R is released from the solid support. Thus, an advantage of the constructs of the present invention is that they can be used both at an experimental level to optimise a particular process step, and also at a preparative level without modifying the linker groups.

In one embodiment of the invention, the fragments Fr, F^{a} or F^{b}, and more preferably the spacer group A contains an alkylene diamine group or aminoalkoxy group. The precise size of the alkylene group and its degree of substitution is not currently considered to be important, but particular examples are ethylene or propylene diamine or aminoalcohol groups which may be substituted or unsubstituted. The alkylene diamine group or aminoalcohol typically contains a peak splitting isotopic label as hereinbefore defined. The two amino groups can each be bonded to respectively the first and second linker groups. In order to increase the mass of the spacer group A, or change its properties, the alkylene diamine group can be substituted by an aryl group, or example an N-aryl group such as an N-benzyl group, or the alkylene chain can be substituted (for example with one or more fluorine atoms to alter the volatility of the analytical fragment). The N-aryl group may optionally be substituted with one or more substituent groups.

Where, as is preferred, the spacer group contains one or more mass-spectral peak splitting isotopic labels, these can be located either in the alkylene chain or in a substituent group attached to the alkylene chain. Thus, for example, an N-benyl group bonded to one of the two amino groups in an alkylenediamine can have a methylene group which is substituted with the peak splitting atom deuterium. Alternatively, an aryl ring (e.g. an N-benzyl group) present in a substituent on the alkylene diamine can be substituted with a peak splitting bromine atom, one particular example of an aryl group being an N-*o*-bromobenzyl group.

The fragments Fr, F^{a} or F^{b}, and in particular the spacer group A, as well as providing a means of identification using mass spectrometry, can also be provided with one or more additional sensitisers to allow characterisation by other analytical techniques. For example, the spacer group can contain a chromophore to allow characterisation by ultra violet (u.v.) or fluorescence spectroscopy.

Although alkylene diamine and amino alcohol groups are given as specific examples of spacer groups, alternative spacer groups can be used. For example, the spacer groups can be formed from hydrocarbon chains containing up to thirty or more carbon atoms in the chain which can be optionally interrupted with one or more heteroatoms such as oxygen, nitrogen or sulphur. As a further alternative, the spacer can be for example a peptide chain containing one or more amino acids. The precise nature and length of the spacer is not currently considered to be important provided that the spacer does not interfere with the chemistry of the construct.

The solid support Q can be any type of solid support suitable for use in solid phase synthesis, and in particular combinatorial chemistry. Thus, purely by way of example, the solid support can take the form of beads, a solid surface, solid substrates, particles. pellets, discs, capillaries, hollow fibres, needles, solid fibres, or organic or inorganic gels such as silica gels, and insoluble organic particles such as particles formed from fullerenes.

Examples of beads are polymeric beads such as cellulose beads or resin beads, particular examples of materials from which resin beads can be prepared including functionalised polymer resins such as polystyrene resins, polyacrylamide resins and dimethylacrylamide resins. Examples of suitable supports are listed in *The Combinatorial Index* by Barry A. Bunin, referred to above.

In another aspect, the invention provides a method of analysing the constructs hereinbefore defined; the method comprising cleaving the groups Y¹ and Y², and optionally the group Y^{a} to release the chemical fragment Fr (and optionally the fragments F^{a} and F^{b} and then subjecting the chemical fragments Fr, and F^{a} to mass spectrometry, e.g. electrospray mass spectrometry.

The analysis of the fragment Fr provides information on the reaction history of the construct. Thus, by mass spectrometric analysis, it can readily be determined whether or not the desired substrate has been formed in a given reaction sequence. Analysis of the fragment Fr can therefore be used not only to characterise the substrate or product of the solid phase reaction sequence, but also to follow the progress of the reactions.

In a further aspect, the invention provides intermediate chemical constructs for use preparing a chemical construct as hereinbefore defined, the intermediate constructs having the formulae Y¹'-Q-Y²', RY¹-Q-Y²'and Y¹'-Q-Y²R wherein Y¹' and Y²' are reactive or protected form of the group Y; and R, Q and Y are as hereinbefore defined.

In a still further aspect, the invention provides intermediate constructs of the formulae L²'-A-L¹-Q-L¹-A^{p}, R-L²-A-L¹-Q-L¹-A^{p}, L³'-A-L¹-Q-L¹-A^{p}, R-L³-A-L¹-Q-L¹-A^{p}, R-L³-A-L¹-Q-L¹-A-L²', L³'-A-L¹-Q-L¹-A-L²-R, wherein L¹', L²' and L³' are reactive or protected forms of the linker groups L¹, L² and L³'L hereinbefore defined, A^{p} is a reactive or protected form of the spacer group A containing a peak splitting isotopic label, and Q, R, A, L¹, L² and L³ are as hereinbefore defined. In a particular embodiment, the group A^{p} has the formula NH-Alk-NX¹ wherein Alk is an alkylene group and X¹ is hydrogen or an aralkyl group. The intermediate construct is preferably isotopically labelled with a peak splitting combination of atoms such as ¹H/H² (D), ⁷⁹Br/⁸¹Br, ¹²C/¹³C, ¹⁴N/¹⁵N and ¹⁶O/¹⁸O.

In each of the aforementioned intermediates, the solid support can have optionally bonded thereto a coding tag sequence L¹-A-Tag and/or a substrate-based tag sequence R - A - L¹ -, and precursor forms thereof such as L¹-A^{p}.

The invention will now be illustrated but not limited by reference to the following examples.

### Brief Description of the Figures

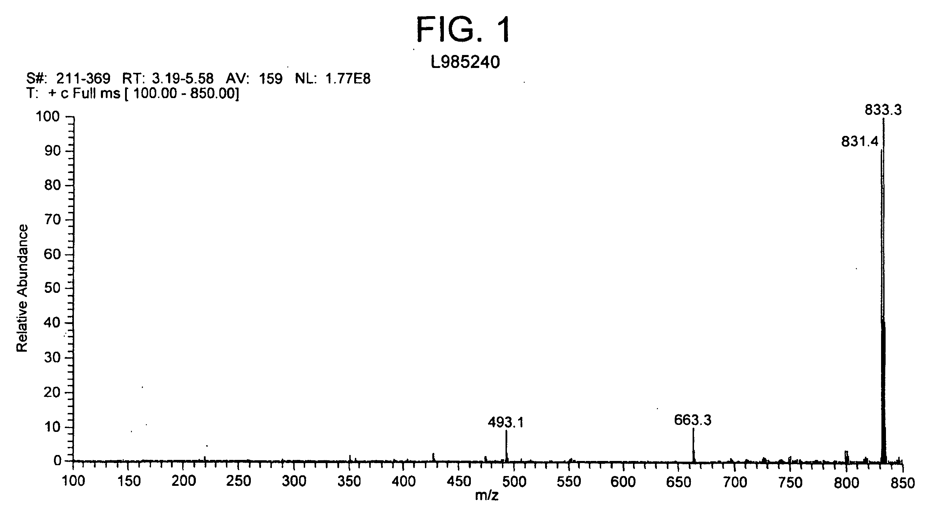

Figure 1 illustrates the mass spectrum of the product of the cleavage, by oxidation and nucleophilic displacement, of a construct of the formula Q-L¹-A-L²-R wherein R is a model compound benzamide, L¹ is a thiopyrimidine linker group, A is an N-bromobenzyl substituted ethylene diamine "peak splitting group" and L² is a linker group which is cleavable under photochemical conditions.

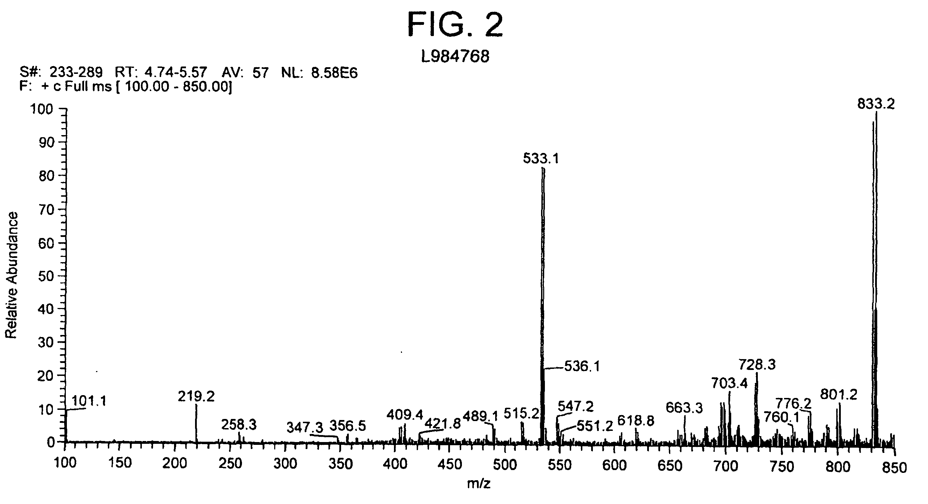

Figure 2 illustrates the mass spectrum of the construct of Figure 1 following photochemical cleavage of the linker L² and subsequent oxidation / nucleophilic displacement at linker L¹.

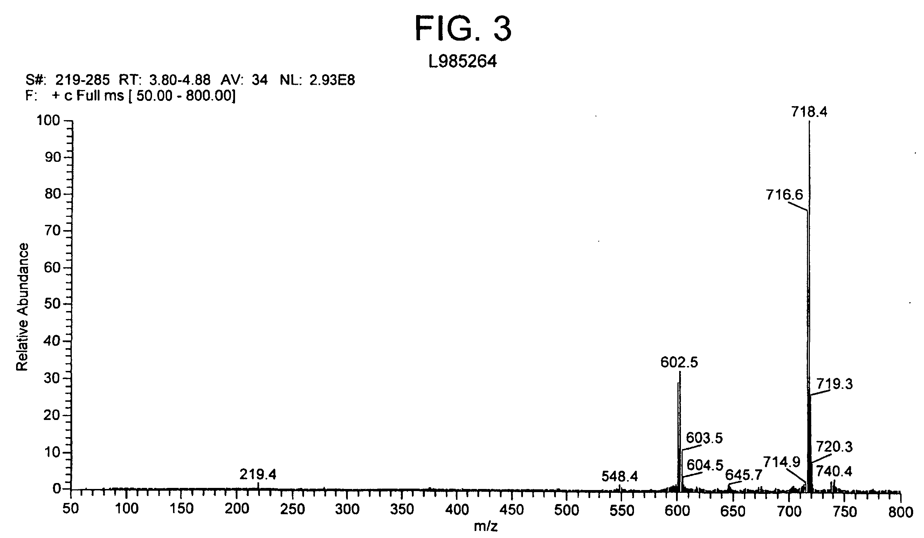

Figure 3 illustrates the mass spectrum of the product of the cleavage, by oxidation and nucleophilic displacement, of a construct of the formula Q-L¹-A-Tag wherein L¹ is a thiopyrimidine linker group, A is the same peak splitting group as is present in the construct of Figure 1, and Tag is a coding sequence consisting of a tripeptide.

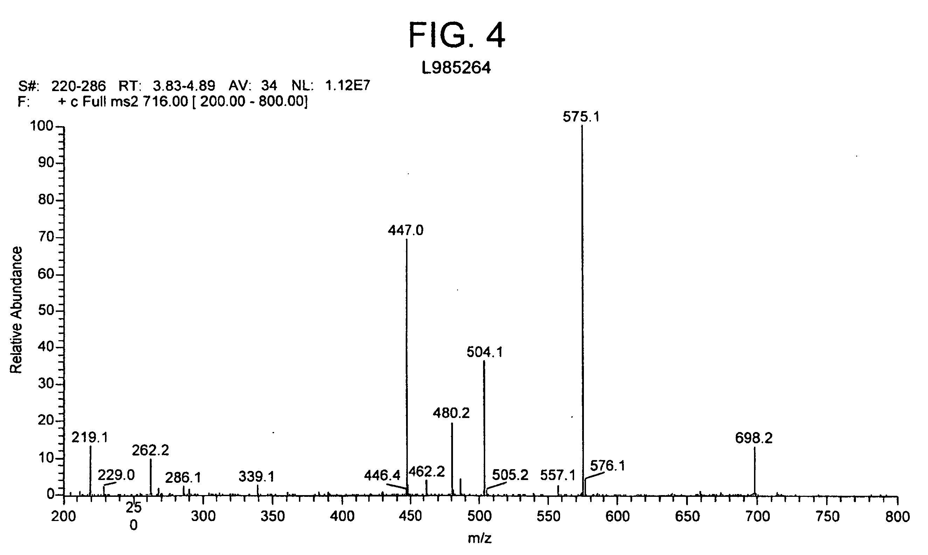

Figure 4 illustrates the mass spectrum of the cleavage product of Figure 3 but wherein the conditions within the mass spectrometer have been adjusted to cause fragmentation of the molecular ion at 716 a.m.u. in order to produce peaks due to constructs in which one or more amino acids have been lost from the molecular ion.

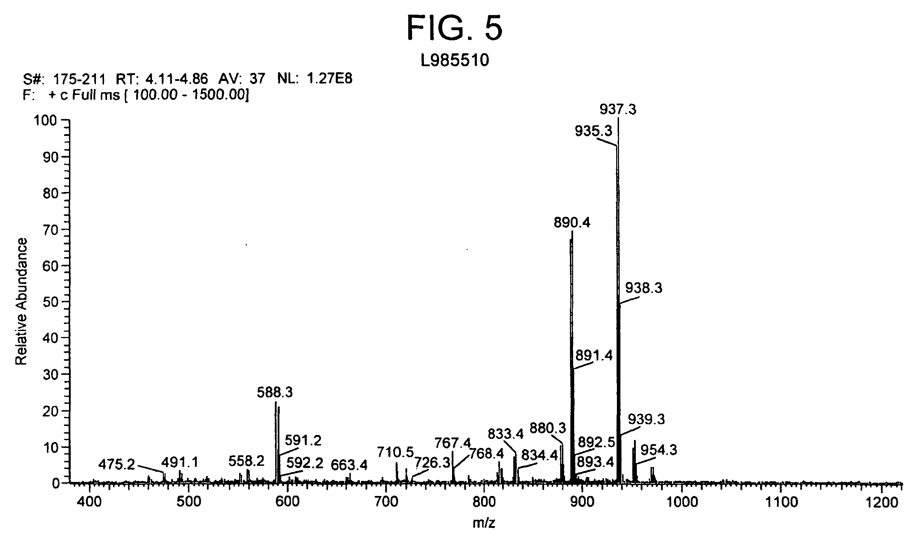

Figure 5 illustrates the mass spectrum of the product of the cleavage, by oxidation and nucleophilic displacement, of a construct of the formula: 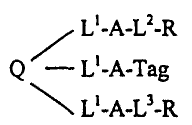 wherein "Tag" represents an allyloxyglycine code group, L¹, L² and A are the same as for the constructs of Figures 1 to 4 and L³ is a linker of the "Rink" or "Knorr" type which is cleavable by trifluoroacetic acid.

### EXAMPLE 1

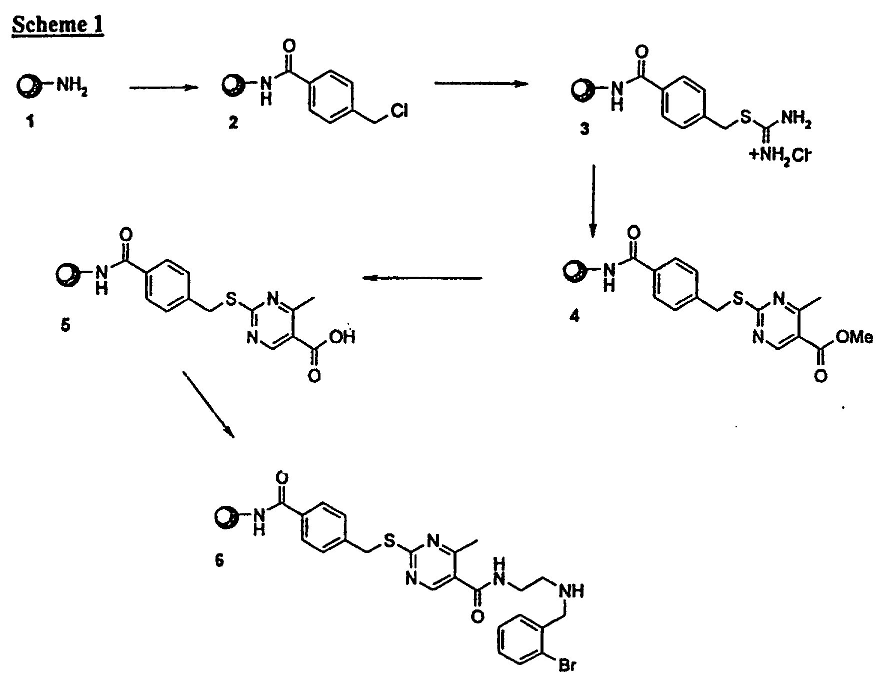

### Experimental for Scheme 1

A solution of 4-(chloromethyl)benzoic acid (0.27 g, 1.6 mmol), Benzotriazole-1-yloxy-tris-pyrrolidino-phosphonium hexafluorophosphate (PyBOP)® (0.83 g, 1.6 mmol) and diisopropylethylamine (0.56 ml, 3.2 mmol) in dimethylformamide (8 ml) was shaken for 20 minutes and was then added to TentaGel™ NH2 (Rapp Polymere) resin **1** (1 g, 032 mmol). The resulting mixture was shaken for 5 hours, following which the resin was drained and washed with dimethylformamide, dichloromethane, diethyl ether, dichloromethane and finally diethyl ether . The resin (**2**) was then dried *in vacuo*.

A slurry of thiourea (0.58 g, 7.7 mmol) in dioxane (4 ml) and ethanol (1 ml) was added to Resin **2** (1 g, 0.32 mmol) and the whole heated at 80 °C for 16 hours. The resin was drained and washed with dimethylformamide (x 5), dichloromethane (x 5), diethyl . ether, dichloromethane and finally diethyl ether to give resin **3**. The resin was then dried *in vacuo*.

A solution of methyl-2-(dimethylaminomethylene)-3-oxobutanoate (0.17 g, 0.96 mmol) and triethylamine (65 ml, 0.48 mmol) in dimethylformamide (10 ml) was added to resin **3** (1 g, 0.31 mmol) and the mixture was heated at 80 °C for 16 hours. The resin was then drained and washed with dimethylformamide, dichloromethane, diethyl ether, dichloromethane, and finally diethyl ether. The washed resin (**4**) was dried *in vacuo*.

Resin **4** (0.5 g, 0.15 mmol) was then treated with a 1:1 mixture of tetrahydrofuran and 2N aqueous sodium hydroxide (4 ml) and shaken for 2 hours. The resin was then drained and washed with tetrahydrofuran/H₂O, 10% acetic acid /tetrahydrofuran, tetrahydrofuran /H₂O, tetrahydrofuran, dimethyl-formamide, dichloromethane, diethyl ether, dichloromethane, diethyl ether, and then dried *in vacuo* to give resin **5**.

A solution of PyBOP® (24 mg, 0.045 mmol) and diisopropylethylamine (16 ml, 0.09 mmol) in dimethylformamide (1 ml) was added to resin **5** (50 mg, 0.015 mmol) followed by a solution of 1-*tert*-butoxycarbonyl-1-(*o*-bromobenzyl)-diaminoethane (15 mg, 0.045 mmol) in dimethylformamide (1 ml) and the mixture was shaken for 3 days. The resin was drained and washed with dimethylformamide, dichloromethane, diethyl ether, dichloromethane, and finally diethyl ether. A solution of 95% aqueous trifluoroacetic acid in dichloromethane (20%, 1 ml) was then added to the resin and the mixture was shaken for 2 hours. The resin was drained and washed with dimethylformamide, dichloromethane, diethyl ether, dichloromethane, and then shaken with 10% diisopropylethylamine in dimethylformamide for 10 minutes. The resin was drained and washed with dimethylformamide, dichloromethane, diethyl ether, dichloromethane, and finally diethyl ether. The resin (**6**) was then dried *in vacuo*.

### EXAMPLE 2

### Experiment to Demonstrate Residual Drug on Construct after photolysis

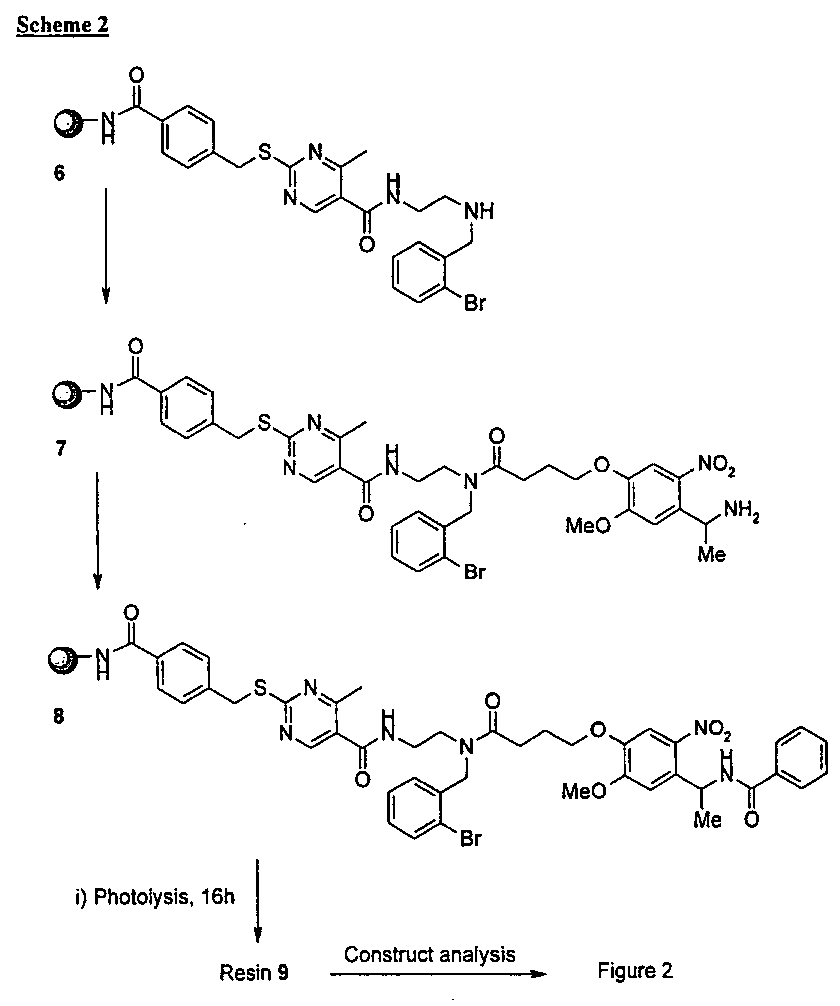

### Experimental for Scheme 2

A solution of 4-[4-(1-(9-fluorenylmethoxycarbonylamino)ethyl)-2-methoxy-5-nitrophenoxy)butanoic acid (23 mg, 0.045 mmol), diisopropylethylamine (16 ml, 0.09 mmol) and 2-(1H-9-azabenzotriazole-1-yl)-1,1,3,3-tetramethyluronium hexafluorophosphate (17 mg, 0.045 mmol) in dimethylformamide (2 ml) was added to resin **6** (50 mg, 0.015 mmol) and the whole was shaken for 3 hours. The resin was drained and washed with dimethylformamide, dichloromethane, diethyl ether, dichloromethane, and finally diethyl ether. The resin was then treated with 20% piperidine in dimethylformamide (3 ml) and the whole was shaken for 1 hour The resin (**7**) was then drained and washed with dimethylformamide, dichloromethane, diethyl ether, dichloromethane, and finally diethyl ether and dried *in vacuo.*

A solution of benzoic acid (11 mg, 0.09 mmol), diisopropylethylamine (16 ml, 0.09 mmol) and 2-(1H-9-azabenzotriazole-1-yl)-1,1,3,3-tetramethyluronium hexafluorophosphate (17 mg, 0.045 mmol) in dimethylformamide (2 ml) was added to resin **7** and the mixture was shaken for 2 hours. The resin (**8**) was then drained and washed with dimethylformamide, dichloromethane, diethyl ether, dichloromethane, and finally diethyl ether.

Analysis of the product of the synthesis was carried out by subjecting the resin-bound construct to thiopyrimidine cleavage conditions (described below), following which the construct was analysed by mass spectrometry. The mass spectrum of the construct is shown in figure **1**.

A solution of 0.2% Hydrazine hydrate in dimethylsulphoxide (0.1 ml) was added to resin 9 (2 mg, 0.6 mmol) and the suspension subjected to photolysis for 16 hours. The photolysed resin (**9**) was then drained and washed with dimethylsulphoxide, dichloromethane, diethyl ether, dichloromethane, and finally diethyl ether and dried *in vacuo*. The washed resin was subjected to pyrimidine cleavage conditions (described below) and the resulting cleavage product was analysed by mass spectrometry. The mass spectrum is shown in figure **2**.

### General method of Construct Analysis of Resins via the Pyrimidine Linker.

A small sample of resin (ca. 0.5 mg) is treated with 0.01M m-chloroperbenzoic acid (0.5 ml) for 2 hours. The resin is then drained and washed with dichloromethane, diethyl ether and dichloromethane. The resin is then treated with 0.02 M N-methylpiperazine in dimethylformamide (50 ml) and the mixture is shaken for 12 hours. The solution is then removed and analysed by mass spectrometry.

### EXAMPLE 3

### Identification of a sensitised sequence of amino acids

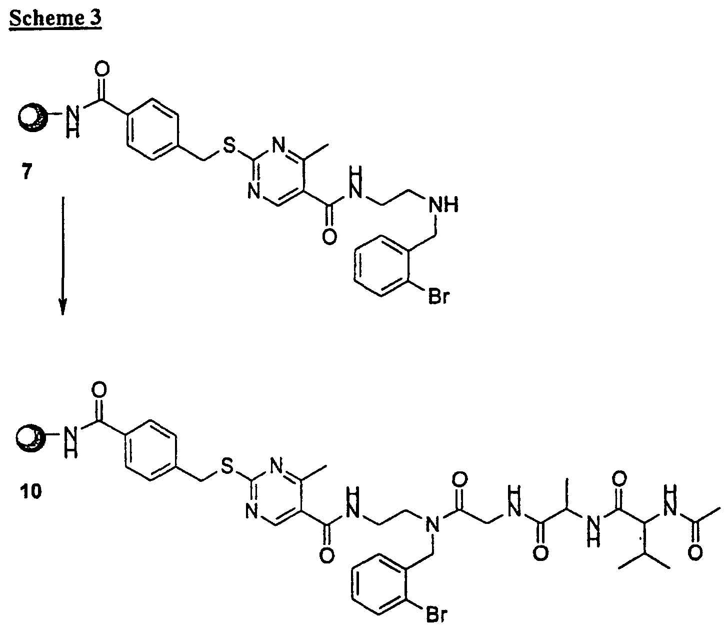

### Experimental for Scheme 3

Resin **10** was prepared by reacting resin **7** with the desired 9-fluorenylmethoxycarbonyl protected amino acids followed by deprotection, and then repeated coupling to the next amino acid and deprotection (see general method). The terminal amino group was then acylated using acetic anhydride (see method below).

The resulting resin was subjected to pyrimidine cleavage conditions (described above) followed by mass spectroscopic analysis. The mass spectrum is shown in Figure **3**. Having identified the molecular ion at mass 716, the mass spectrum was run again at a higher voltage in order to bring about fragmentation and sequential cleavage of the three amino acids. The mass spectrum of the fragmented construct is shown in Figure **4**.

### General/Typical method for coupling amino acids to the linker

A solution of the 9-fluorenylmethoxycarbonyl protected amino acid (0.14 mmol, 10 Eq), diisopropylcarbodiimide (22 ml, 0.14 mmol, 10 Eq) and N-hydroxybenzotriazole (19 mg, 0.14 mmol, 10 Eq) in dimethylformamide (1ml) was added to required resin **7** (30 mg, ca. 0.014 mmol). The mixture was then shaken for 5 hours. The resin was then drained and washed with dichloromethane, dimethylformamide, dichloromethane, diethyl ether, dichloromethane, and finally diethyl ether. The 9-fluorenylmethoxycarbonyl protecting groups were then removed by the treatment of the resins with 20% piperidine solution in dimethylformamide (2 ml) and shaking for 30 minutes. The resin was then drained and washed with dichloromethane, dimethylformamide, dichloromethane, diethyl ether, dichloromethane, and finally diethyl ether. The washed resin was dried *in vacuo*.

### Acetylation of the dipeptides

A solution of acetic anhydride (15 ml, 0,14 mmol) in dichloromethane (1 ml) was added to the resins (30 mg, ca. 0.014 mmol) followed by 4-dimethylaminopyridine (1 mg, 0.008 mmol) and the mixture was shaken for 2 hours. The resin was then drained and washed with dichloromethane, dimethylformamide, dichloromethane, diethyl ether, dichloromethane, and finally diethyl ether. The washed resin was dried *in vacuo*.

### EXAMPLE 4

### Preparation of Differential Release Resin 11

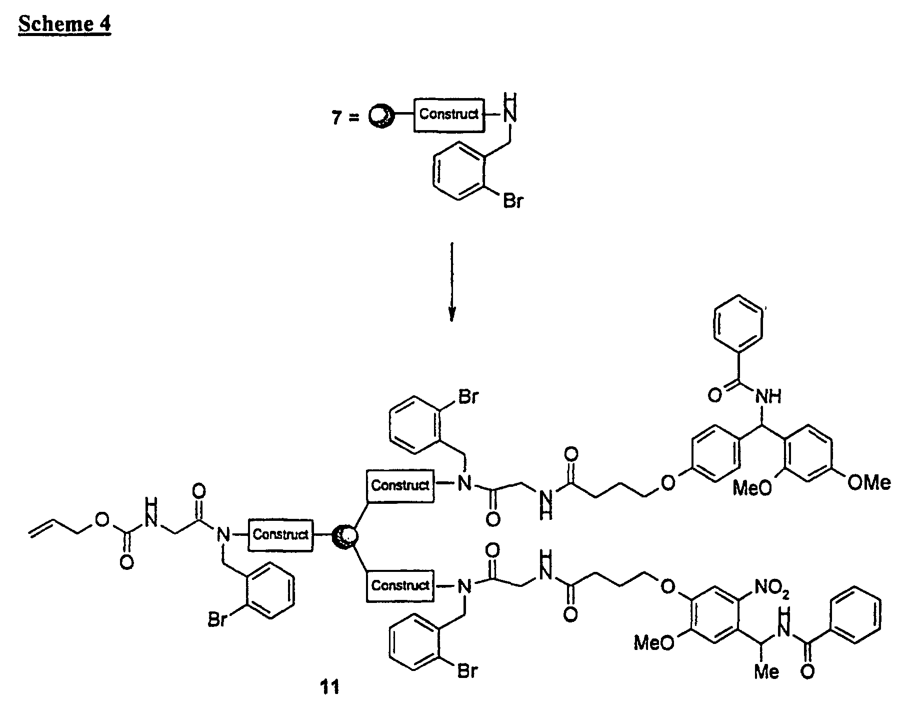

### Experimental for Scheme 4

Resin **7** (0.1 g, 0.03 mmol) was treated with a solution of 9-fluorenylmethoxycarbonyl glycine (36 mg, 0.12 mmol), N-allyloxycarbonyl glycine (5.2 mg, 0.03 mmol), PyBOP (78 mg, 0.15 mmol) and diisopropylethylamine (52 ml, 0.3 mmol) in dimethylformamide (1 ml) and the resulting mixture was shaken for 3 hours. The resin was then drained and washed with dimethylformamide, dichloromethane, diethyl ether, dichloromethane, and finally diethyl ether and dried *in vacuo*.

The resin thus produced was treated with 20% piperidine in dimethylformamide (2 ml) and shaken for 30 minutes before draining and washing as described above. The resin was then treated with a solution of 4-[4-(1-(9-fluorenylmethoxycarbonylamino)ethyl)-2-methoxy-5-nitrophenoxy)butanoic acid (39 mg, 0.075 mmol), p-[(R,S)-α-[1-(9H-fluoren-9-yl)-methoxyformamido-]-2,4-dimethoxy-benzyl]-phenoxybutanoic acid (43 mg, 0.075 mmol), PyBOP (78 mg, 0.15 mmol) and diisopropylethylamine (52 ml, 0.3 mmol) in dimethylformamide (Iml) and the mixture was shaken for 3 hours. The resin was then drained and washed with dimethylformamide, dichloromethane, diethyl ether, dichloromethane, and finally diethyl ether and dried *in vacuo*.

This resin was then treated with 20% piperidine in dimethylformamide (2 ml) and shaken for 30 minutes. The resin was then drained and washed as above. The resin was then treated with a solution of benzoic acid (18 mg, 0.15 mmol), 2-(1H-9-azabenzotriazole-1-yl)-1,1,3,3-tetramethyluronium hexafluorophosphate (57 mg, 0.15 mmol) and diisopropylethylamine (52 ml, 0.3 mmol) in dimethylformamide (1 ml) and the whole was shaken for 2 hours. This resin was then drained and washed as above.

The product resin was subjected to pyrimidine cleavage conditions (described above) followed by mass spectroscopic analysis, and the mass spectrum thus obtained is shown in figure **4**.

It will readily be apparent that numerous modifications and alterations could be made to the constructs described in the examples above without departing from the principles underlying this invention, and all such modifications and alterations are intended to be embraced by the claims appended hereto.

## Claims

1. A chemical construct for use in solid phase synthesis comprising a solid support Q having linked thereto groups Y¹R and Y²R; wherein R is a substrate or a coding tag and the groups Y¹ and Y² are connecting groups each having a first cleavage site, at least one of Y¹ and Y² having a second cleavage site located between the first cleavage site and group R, the first cleavage site being orthogonally and selectively cleavable with respect to the second cleavage site, and, when both groups Y¹ and Y² contain a second cleavage site, the second cleavage site in Y¹ being selectively and orthogonally cleavable with respect to the second cleavage site in Y²; the second cleavage site being cleavable to release the substrate; and the first cleavage site being selectively cleavable to release a fragment Fr comprising the substrate R and at least a portion of the connecting group Y; and wherein:
(i) the chemical fragment Fr contains a sensitising group G which sensitises the chemical fragment Fr to instrumental, e.g. mass spectroscopic analysis and/or:
(ii) the fragment Fr contains a means for imparting a characteristic signature to the mass spectrum of the fragment.

2. A chemical construct according to claim 1 wherein at least one group R is a substrate.

3. A chemical construct according to claim 1 comprising a solid support Q having linked thereto groups Y¹R and Y²R; wherein R is a substrate (such as a drug molecule) and the groups Y¹ and Y² are connecting groups each having first and second cleavage sites which are orthogonally and selectively cleavable, the second cleavage site in Y¹ being selectively and orthogonally cleavable with respect to the second cleavage site in Y²; the second cleavage site being cleavable to release the substrate; and the first cleavage site being located at a position between the second cleavage site and the solid support and being selectively cleavable to release a fragment Fr comprising the substrate R and at least a portion of the connecting group Y; and wherein:
(i) the chemical fragment Fr contains a sensitising group G which sensitises the chemical fragment Fr to instrumental, e.g. mass spectroscopic analysis and/or.
(ii) the fragment Fr contains a means for imparting a characteristic signature to the mass spectrum of the fragment.

4. A chemical construct according to claim 1 having the formula:

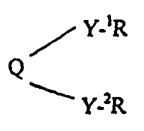

5. A chemical construct according to any one of the preceding claims wherein each solid support contains a coding tag or coding sequence which encodes information indicative of at least part of the synthesis history of the construct.

6. A chemical construct according to claim 5 wherein the coding tag is a coding sequence linked to the solid support.

7. A chemical construct according to claim 6 wherein the coding sequence is linked to the solid support by means of a connecting group Y^{a} having a cleavage site cleavable to release a fragment F^{a} from the solid support, the fragment F^{a} comprising the coding sequence and optionally at least a portion of the connecting group Y^{a}.

8. A chemical construct according to claim 7 wherein:
(i) the chemical fragment F^{a} contains a sensitising group G which sensitises the chemical fragment F^{a} to instrumental, e.g. mass spectroscopic analysis and/or:
(ii) the fragment F^{a} contains a means for imparting a characteristic signature to the mass spectrum of the fragment.

9. A chemical construct according to claim 7 wherein the cleavage site of group Y^{a} is cleavable under conditions corresponding to those needed to cleave the first cleavage sites in the groups Y¹R and Y²R.

10. A chemical construct according to claim 8 wherein the chemical fragment F^{a} contains a sensitising group G which sensitises the chemical fragment F^{a} to instrumental, e.g. mass spectroscopic analysis.

11. A chemical construct according to any one of the preceding claims wherein a proportion of the total substrate R in the construct is linked to the solid support by means of a connecting group Y^{b} having a cleavage site which is cleavable to release a fragment F^{b} from the solid support, the fragment F^{b} comprising the substrate R and at least a portion of the connecting group Y^{b}; the connecting group Y^{b} not being cleavable to release substrate R under conditions effective to cleave the second cleavage sites in the groups Y¹R and Y²R and wherein:
(i) the chemical fragment F^{b} contains a sensitising group G which sensitises the chemical fragment F^{b} to instrumental, e.g. mass spectroscopic analysis and/or:
(ii) the fragment F^{b} contains a means for imparting a characteristic signature to the mass spectrum of the fragment.

12. A chemical construct according to any one of the preceding claims wherein the sensitising group G is generated by cleavage at the first cleavage site of the group Y¹ or Y² or, when present, Y^{a} or the said cleavage site of Y^{b}.

13. A chemical construct according to any one of the preceding claims wherein the sensitising group G is a basic amino group or a carboxylate group, preferably a basic amino group.

14. A chemical construct according to claim 13 wherein the sensitising group is a primary amino group, a secondary amino group, or a tertiary amino group.

15. A chemical construct according to claim 14 wherein the sensitising group is a tertiary amino group selected from cyclic amino groups such as piperidino, piperazino (e.g.N-methylpiperazino), pyrrolidino, or morpholino, piperidino.

16. A construct according to any one of the preceding claims wherein the fragment Fr and (where present) optionally the fragment F^{a} and (where present) optionally the fragment F^{b} contain a means for imparting a characteristic signature to the mass spectrum of the fragment.

17. A construct according to claim 16 wherein the signature is provided by incorporating into the fragment a "peak splitting" isotopic label comprising at least one atom that exists in a number of stable isotopic forms.

18. A chemical construct according to claim 17 wherein the fragments Fr and F^{a}, and (where present) optionally F^{b} are labelled differently so as to produce different characteristic signatures.

19. A chemical construct according to claim 17 or claim 18 wherein the isotopic label comprises an atom or atoms selected from ¹H/H² (D), ⁷⁹Br/⁸¹Br, ¹²C/¹³C, ¹⁴N/¹⁵N and ¹⁶O/¹⁸O.

20. A chemical construct according to any one of claims 17 to 19 wherein the isotopic label(s) is/are located between the first and second cleavage sites of the groups Y¹ and Y².

21. A chemical construct as defined in any one of the preceding claims wherein the first and second cleavage sites in the groups Y¹ are defined by first and second linker groups L¹ and L², first and second cleavage sites in the group Y² are defined by first and second linker groups L¹ and L³, the cleavage site in the group Y^{a} (where present) is defined by a linker group L^{a} and the cleavage site in the group Y^{b} (where present) is defined by a linker group L^{b}.

22. A chemical construct according to claim 21 wherein the linker groups L^{a} and L^{b} correspond to L¹.

23. A chemical construct according to claim 21 or claim 22 wherein a spacer group A is interposed between each pair of first and second linker groups, or between the linker group L^{a} and the coding tag, or between the linker group L^{b} and the substrate R, the spacer group A containing an isotopic peak splitting label.

24. A chemical construct according to any one of the preceding claims having a formula selected from the group consisting of: 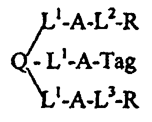 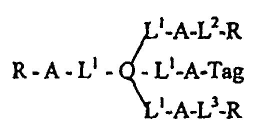 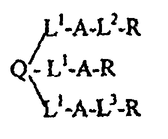 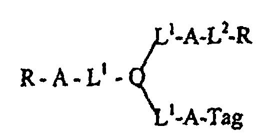
Tag-A-L¹-Q-L¹-A-L²-R
R-A-L¹-Q-L¹-A-L²-R 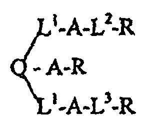 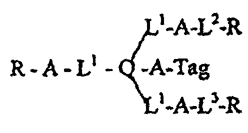 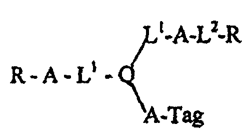 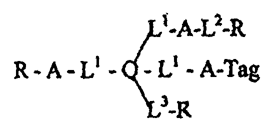 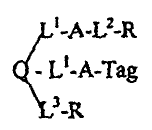 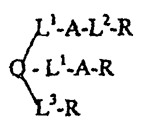
R-L³-Q-L¹-A-L²-R 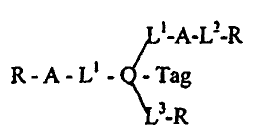
wherein L¹, L², L³, A and R are as defined in any one of the preceding claims and "Tag" represents a coding sequence.

25. A construct as claimed in any one of claims 1 to 24 for use in a tiered release method of screening, the construct having the formula Tag - A - L¹ - Q - L¹ - A -L² - R wherein Tag, A, L¹, Q,L² and R are as defined in any one of the preceding claims.

26. A chemical construct according to any one of the preceding claims wherein the orthogonally cleavable cleavage sites can be cleaved by a reactions selected from acid catalysed cleavage, base catalysed cleavage, oxldative cleavage, reductive cleavage, nucleophilic displacement, electrophilic displacement, and thermal, photochemical and enzymatic cleavage.

27. Intermediate chemical constructs for use preparing a chemical construct as defined in any one of the preceding claims, the intermediate constructs having the formulae Y¹-Q-Y², RY¹-Q-Y² and Y¹-Q-Y²R wherein Y¹ and Y² are reactive or protected forms of the group Y; and R, Q and Y are as defined in any one of the preceding claims.

28. Intermediate constructs of the formulae L²-A-L¹-Q-L¹-A^{p}, R-L²-A-L¹-Q-L¹-A^{p}, L³'-A-L¹-Q-L¹-A^{p}, R-L³-A-L¹-Q-L¹-A^{p}, R-L³-A-L¹-Q-L¹-A-L² and L³-A-L¹-Q-L¹-A-L²-R wherein L¹, L² and L³ are reactive or protected forms of the linker groups L¹, L² and L³, A^{p} is a reactive or protected form of the spacer group A containing a peak splitting isotopic label, and Q, R, A, L¹, L² and L³ are as defined in any one of the preceding claims.

29. An intermediate construct according to claim 28 wherein the group A^{p} has the formula NH-Alk-NX¹ wherein Alk is an alkylene group and X¹ is hydrogen or an aralkyl group.

30. An intermediate construct according to claim 28 or claim 29 wherein the solid support has bonded thereto a coding tag sequence L¹-A-Tag and/or a sequence R - A - L¹ -, or a precursor form thereof.

31. A differential release method of assaying a chemical library for biological activity, the method comprising:
(i) subjecting a construct comprising a solid support Q having linked thereto groups Y¹R and Y²R as defined in any one of the preceding claims to cleavage conditions effective to release substrate R from the group Y¹R;
(ii) testing the substrate R released from the group Y¹R in a biological assay;
(iii) subsequently subjecting the construct to cleavage conditions effective to release substrate R from the group Y²R; and
(iv)) testing the substrate R released from the group Y²R in a biological assay.

32. A tiered release method of assaying a chemical library for biological activity, the method comprising:
(i) subjecting a construct as claimed in any one of claims 1 to 26 to cleavage conditions effective to release a first portion of the substrate R from the group Y¹R;
(ii) testing the first portion of substrate R released from the group Y¹R in a biological assay;
(iii) subjecting the construct to cleavage conditions effective to release a second portion of the substrate R from the group Y¹R; and
(iv) testing the second portion of substrate R released from the group Y¹R in a biological assay.

33. A method of determining the identity of a substrate R linked to a solid support Q of a construct as claimed in any one of claims 7 to 26 by mass spectrometric means; the solid support Q having a coding sequence attached thereto by means of a connecting group Y^{a} having a cleavage site cleavable to release a fragment F^{a} from the solid support, the fragment F^{a} comprising the coding sequence and at least a portion of the connecting group Y^{a}, wherein (i) the chemical fragment F^{a} contains a sensitising group G which sensitises the chemical fragment F^{a} to mass spectroscopic analysis;
the coding sequence comprising a sequence of coding groups the nature and order of which is indicative of the identity of the substrate R;
the method comprising cleaving the connecting group Y^{a} so as to release the fragment F^{a} from the solid support; subjecting the fragment Y^{a} to mass spectrometry under conditions effective to bring about mass spectral fragmentation of the coding group and the formation of mass spectral fragment Ions corresponding to the loss of one or more coding groups from the coding sequence, and thereafter correlating mass spectral peaks of the mass spectral fragment ions with the molecular ion of the fragment Y^{a} to identify the sequence of the individual coding groups.

34. A method according to claim 33 wherein the fragment F^{a} contains a means for imparting a characteristic signature to the mass spectrum of the fragment.

35. A method of identifying a pharmaceutically useful substrate comprising preparing a library containing a plurality of chemical constructs as defined in any of the preceding claims, and subjecting the library to biological testing to identify biologically active substrates.

36. A method according to claim 35 that includes the further step of formulating a biologically active substrate thus identified with a pharmaceutically acceptable carrier to form a pharmaceutical composition.

## Patentansprüche

1. Chemisches Konstrukt zur Verwendung in der Festphasensynthese, umfassend einen festen Träger Q mit daran gebundenen Resten Y¹R und Y²R; wobei R ein Substrat oder eine Kodierungsmarkierung ist und die Reste Y¹ und Y² Verbindungsgruppen mit jeweils einer ersten Spaltungsstelle sind, wobei mindestens einer der Reste Y¹ und Y² eine zweite Spaltungsstelle aufweist, die sich zwischen der ersten Spaltungsstelle und dem Rest R befindet, wobei die erste Spaltungsstelle orthogonal und selektiv spaltbar in Bezug auf die zweite Spaltungsstelle ist, und, werm beide Reste Y¹ und Y² eine zweite Spaltungsstelle enthalten, die zweite Spaltungsstelle in Y¹ selektiv und orthogonal in Bezug auf die zweite Spaltungsstelle in Y² spaltbar ist; die zweite Spaltungsstelle spaltbar ist, wobei das Substrat freigesetzt wird, und die erste Spaltungsstelle selektiv spaltbar ist, wobei ein Fragment Fr freigesetzt wird, das das Substrat R und mindestens einen Teil der Verbindungsgruppe Y umfasst; und wobei:
(i) das chemische Fragment Fr eine Sensibilisierungsgruppe G enthält, die das chemische Fragment Fr für instrumentale, z.B. Massenspektroskopie-Analyse sensibilisiert und/oder:
(ii) das Fragment Fr ein Mittel enthält, um dem Massenspektrum des Fragments eine charakteristische Signatur zu verleihen.

2. Chemisches Konstrukt nach Anspruch 1, in dem mindestens ein Rest R ein Substrat ist.

3. Chemisches Konstrukt nach Anspruch 1, umfassend einen festen Träger Q mit daran gebundenen Resten Y¹R und Y²R; wobei R ein Substrat (wie ein Arzneistoffmolekül) ist und die Reste Y¹ und Y² Verbindungsgruppen mit jeweils einer ersten und zweiten Spaltungsstelle sind, die orthogonal und selektiv spaltbar sind, wobei die zweite Spaltungsstelle in Y¹ selektiv und orthogonal in Bezug auf die zweite Spaltungstelle in Y² spaltbar ist; die zweite Spaltungsstelle spaltbar ist, wobei das Substrat freigesetzt wird, und die erste Spaltungsstelle sich an einer Stelle zwischen der zweiten Spaltungsstelle und dem festen Träger befindet und selektiv spaltbar ist, wobei ein Fragment Fr freigesetzt wird, das das Substrat R und mindestens einen Teil der Verbindungsgruppe Y umfasst; und wobei:
(i) das chemische Fragment Fr eine Sensibilisierungsgruppe G enthält, die das chemische Fragment Fr für instrumentale, z.B. Massenspektroskopie-Analyse sensibilisiert und/oder:
(ii) das Fragment Fr ein Mittel enthält, um dem Massenspektrum des Fragments eine charakteristische Signatur zu verleihen.

4. Chemisches Konstrukt nach Anspruch 1 der Formel:

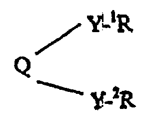

5. Chemisches Konstrukt nach einem der vorstehenden Ansprüche, in dem jeder feste Träger eine Kodierungsmarkierung oder eine Kodierungssequenz enthält, die Information enthält, die ein Hinweis auf mindestens einen Teil der Synthesegeschichte des Konstrukts ist

6. Chemisches Konstrukt nach Anspruch 5, in dem die Kodierungsmarkierung eine an den festen Träger gebundene Kodierungssequenz ist.

7. Chemisches Konstrukt nach Anspruch 6, in dem die Kodierungssequenz an den festen Träger durch eine Verbindungsgruppe Y^{a} mit einer Spaltungsstelle gebunden ist, die unter Freisetzung eines Fragments F^{a} vom festen Träger spaltbar ist, wobei das Fragment F^{a} die Kodierungssequenz und gegebenenfalls mindestens einen Teil der Verbindungsgruppe Y^{a} umfasst.

8. Chemisches Konstrukt nach Anspruch 7, in dem:
(i) das chemische Fragment F^{a} eine Sensibilisierungsgruppe G enthält, die das chemische Fragment F^{a} für instrumentale, z.B. Massenspektroskopie-Analyse sensibilisiert und/oder:
(ii) das Fragment F^{a} ein Mittel enthält, um dem Massenspektrum des Fragments eine charakteristische Signatur zu verleihen.

9. Chemisches Konstrukt nach Anspruch 7, in dem die Spaltungsstelle des Rests Y^{a} unter Bedingungen spaltbar ist, die den zur Abspaltung der ersten Spaltungsstellen in den Resten Y¹R und Y²R erforderlichen entsprechen.

10. Chemisches Konstrukt nach Anspruch 8, in dem das chemische Fragment F^{a} eine Sensibilisierungsgruppe G enthält, die das chemische Fragment F^{a} für instrumentale, z.B. Massenspektroskopie-Analyse sensibilisiert.

11. Chemisches Konstrukt nach einem der vorstehenden Ansprüche, in dem ein Teil des gesamten Substrats R im Konstrukt an den festen Träger mit einer Verbindungsgruppe Y^{b} mit einer Spaltungsstelle gebunden ist, die unter Freisetzung eines Fragments F^{b} vom festen Träger spaltbar ist, wobei das Fragment F^{b} das Substrat R und mindestens einen Teil der Verbindungsgruppe Y^{b} umfasst; wobei die Verbindungsgruppe Y^{b} zur Freisetzung des Substrats R nicht unter Bedingungen spaltbar ist, die zum Abspalten der zweiten Spaltungsstellen in den Resten Y¹R und Y²R wirksam sind; und wobei:
(i) das chemische Fragment F^{b} eine Sensibilisierungsgruppe G enthält, die das chemische Fragment F^{b} für instrumentale, z.B. Massenspektroskopie-Analyse sensibilisiert und/oder:
(ii) das Fragment F^{b} ein Mittel enthält, um dem Massenspektrum des Fragments eine charakteristische Signatur zu verleihen.

12. Chemisches Konstrukt nach einem der vorstehenden Ansprüche, wobei die Sensibilisierungsgruppe G durch Spaltung an der ersten Spaltungsstelle des Rests Y¹ oder Y² oder, wenn vorhanden, Y^{a} oder der Spaltungsstelle von Y^{b} erzeugt wird.

13. Chemisches Konstrukt nach einem der vorstehenden Ansprüche, wobei die Sensibilisierungsgruppe G eine basische Aminogruppe oder eine Carboxylatgruppe, vorzugsweise eine basische Aminogruppe, ist.

14. Chemisches Konstrukt nach Anspruch 13, in dem die Sensibilisierungsgruppe eine primäre Aminogruppe, eine sekundäre Aminogruppe oder eine tertiäre Aminogruppe ist.

15. Chemisches Konstrukt nach Anspruch 14, in dem die Sensibilisierungsgruppe eine tertiäre Aminogruppe, ausgewählt aus cyclischen Aminogruppen, wie Piperidin, Piperazin- (z.B. N-Methylpiperazin-), Pyrrolidin- oder Morpholin-, Piperidingruppen ist.

16. Konstrukt nach einem der vorstehenden Ansprüche, in dem das Fragment Fr und (wenn vorhanden) gegebenenfalls das Fragment F^{a} und (wenn vorhanden) gegebenenfalls das Fragment F^{b} ein Mittel enthalten, um dem Massenspektrum des Fragments eine charakteristische Signatur zu verleihen.

17. Konstrukt nach Anspruch 16, in dem die Signatur durch Einmischen in das Fragment einer "peakauftrennenden" isotopen Markierung bereitgestellt wird, die mindestens ein Atom umfasst, das in einer Reihe von stabilen isotopen Formen existiert.

18. Chemisches Konstrukt nach Anspruch 17, in dem die Fragmente Fr und F^{a} und (wenn vorhanden) gegebenenfalls F^{b} unterschiedlich markiert sind, um so unterschiedliche charakteristische Signaturen zu bewirken.

19. Chemisches Konstrukt nach Anspruch 17 oder Anspruch 18, in dem die isotope Markierung ein Atom oder Atome umfasst, ausgewählt aus ¹H/²H (D), ⁷⁹Br/⁸¹Br, ¹²C/¹³C,¹⁴N/¹⁵N und ¹⁶O/¹⁸O.

20. Chemisches Konstrukt nach einem der Ansprüche 17 bis 19, in dem die isotope(n) Markierung(en) sich zwischen den ersten und zweiten Spaltungsstellen der Reste Y¹ und Y² befindet/befinden.

21. Chemisches Konstrukt nach einem der vorstehenden Ansprüche, in dem die ersten und zweiten Spaltungsstellen in den Resten Y¹ durch die erste und zweite Verbindungsgruppe L¹ und L² festgelegt sind, erste und zweite Spaltungsstellen im Rest Y² durch erste und zweite Verbindungsgruppen L¹ und L³ festgelegt sind, die Spaltungsstelle im Rest Y^{a} (wenn vorhanden) durch eine Verbindungsgruppe L^{a} festgelegt ist und die Spaltungsstelle im Rest Y^{b} (wenn vorhanden) durch eine Verbindungsgruppe L^{b} festgelegt ist.

22. Chemisches Konstrukt nach Anspruch 21, in dem die Verbindungsgruppen L^{a} und L^{b} L¹ entsprechen.

23. Chemisches Konstrukt nach Anspruch 21 oder Anspruch 22, wobei eine Abstandsgruppe A sich zwischen jedem Paar der ersten und zweiten Verbindungsgruppe oder zwischen der Verbindungsgruppe L^{a} und der Kodierungsmarkierung oder zwischen der Verbindungsgruppe L^{b} und dem Substrat R befindet, wobei die Abstandsgruppe A eine isotope peakauftrennende Markierung enthält.

24. Chemisches Konstrukt nach einem der vorstehenden Ansprüche mit einer Formel, ausgewählt aus: 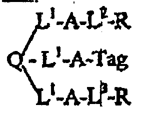 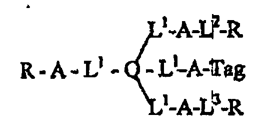 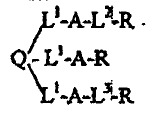 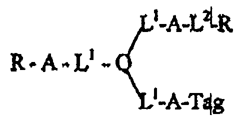
Tag-A-L¹-Q-L¹-A-L²-R
R-A-L¹-Q-L¹-A-L²-R 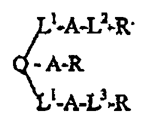 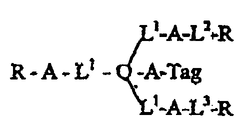 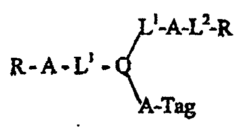 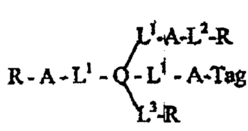 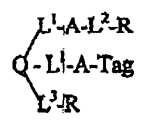 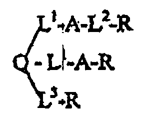
R-L³-Q-L¹-A-L²-R 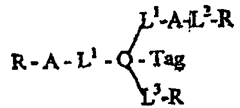
wobei L¹, L², L³, A und R die in einem der vorstehenden Ansprüche angegebene Bedeutung haben und "Tag" eine Kodierungssequenz bedeutet.

25. Konstrukt nach einem der Ansprüche 1 bis 24 zur Verwendung in einem Reihenfreisetzungsverfahren des Screenings, wobei das Konstrukt die Formel Tag-A-L¹-Q-L¹-A-L²-R aufweist, wobei Tag, A, L¹, Q, L² und R die in einem der vorstchenden Ansprüche angegebene Bedeutung haben.

26. Chemisches Konstrukt nach einem der vorstehenden Ansprüche, in dem die orthogonal spaltbaren Spaltungsstellen durch Reaktionen, ausgewählt aus säurekatalysierter Spaltung, basenkatalysierter Spaltung, oxidativer Spaltung, reduktiver Spaltung, nucleophilem Ersetzen, elektrophilem Ersetzen und thermischer, photochemischer und enzymatischer Spaltung, gespalten werden können.

27. Chemische Zwischenprodukt-Konstrukte zur Verwendung bei der Herstellung eines chemischen Konstrukts nach einem der vorstehenden Ansprüche, wobei die Zwischenprodukt-Konstrukte die Formeln Y¹-Q-Y², RY¹-Q-Y² und Y¹-Q-Y²R aufweisen, in denen Y¹ und Y² reaktive oder geschützte Formen des Rests Y sind und R, Q und Y die in einem der vorstehenden Ansprüche angegebene Bedeutung haben.

28. Zwischenprodukt-Konstrukte der Formeln L²-A-L¹-Q-L¹-A^{p}, R-L²-A-L¹-Q-L¹-A^{p}, L³-A-L¹-Q-L¹-A^{p}, R-L³-A-L¹-Q-L¹-A^{p}-, R-L³-A-L¹-O-L¹-A-L² und L³-A-L¹-Q-L¹-A-L²-R, in denen L¹, L² und L³ reaktive oder geschützte Formen der Verbindungsgruppen L¹, L² und L³ sind, A^{p} eine reaktive oder geschützte Form der Abstandsgruppe A ist, die eine peakauftrennende isotope Markierung enthält, und Q, R, A, L¹, L² und L³ die in einem der vorstehenden Ansprüche angegebene Bedeutung haben.

29. Zwischenprodukt-Konstrukt nach Anspruch 28, in dem die Gruppe A^{p} die Formel NH-Alk-NX¹ aufweist, in der Alk ein Alkylenrest ist und X¹ ein Wasserstoffatom oder Aralkylrest ist.

30. Zwischenprodukt-Konstrukt nach Anspruch 28 oder Anspruch 29, in dem der feste Träger daran gebunden eine Kodierungsmarkierungssequenz L¹-A-Tag und/oder eine Sequenz R-A-L¹- oder eine Vorstufenform davon aufweist.

31. Differentialfreisetzungsverfahren zum Prüfen einer chemischen Bibliothek auf biologische Wirksamkeit, wobei das Verfahren umfasst:
(i) Aussetzen eines einen festen Träger Q mit daran gebundenen Resten Y¹R und Y²R umfassenden Konstrukts nach einem der vorstehenden Ansprüche an Spaltungsbedingungen, die wirksam sind, um das Substrat R vom Rest Y¹R freizusetzen;
(ii) Testen des vom Rest Y¹R freigesetzten Substrats R in einem biologischen Test;
(iii) anschließend Aussetzen des Konstrukts an Spaltungsbedingungen, die wirksam sind, um das Substrat R vom Rest Y²R freizusetzen; und
(iv) Testen des vom Rest Y²R freigesetzten Substrats R in einem biologischen Test.

32. Reihenfreisetzungsverfahren zum Prüfen einer chemischen Bibliothek auf biologische Wirksamkeit, wobei das Verfahren umfasst:
(i) Aussetzen eines Konstrukts nach einem der Ansprüche 1 bis 26 an Spaltungsbedingungen, die wirksam sind, einen ersten Teil des Substrats R vom Rest Y¹R freizusetzen;
(ii) Testen des ersten Teils des vom Rest Y¹R freigesetzten Substrats R in einem biologischen Test;
(iii) Aussetzen des Konstrukts an Spaltungsbedingungen, die wirksam sind, um einen zweiten Teil des Substrats R vom Rest Y¹R freizusetzen; und
(iv) Testen des zweiten Teils des vom Rest Y¹R freigesetzten Substrats R in einem biologischen Test.

33. Verfahren zur Bestimmung der Identität eines an einen festen Träger Q eines Konstrukts nach einem der Ansprüche 7 bis 26 gebundenen Substrats R durch massenspektrometrische Verfahren, wobei der feste Träger Q eine Kodierungssequenz daran durch eine Verbindungsgruppe Y^{a} gebunden mit einer Spaltungsstelle aufweist, die unter Freisetzen eines Fragments F^{a} vom festen Träger spaltbar ist, wobei das Fragment F^{a} die Kodierungssequenz und mindestens einen Teil der Verbindungsgruppe Y^{a} umfasst, wobei (i) das chemische Fragment F^{a} eine Sensibilisierungsgruppe G enthält, die das chemische Fragment F^{a} für Massenspektroskopie-Analyse sensibilisiert;
die Kodierungssequenz eine Sequenz von Kodierungsgruppen der Art und Reihenfolge umfasst, die ein Hinweis auf die Identität des Substrats R ist;
das Verfahren das Spalten der Verbindungsgruppe Y^{a}, um so das Fragment F^{a} vom festen Träger freizusetzen; Massenspektrometrie des Fragments Y^{a} unter effektiven Bedingungen, um eine Massenspektralfragmentierung der Kodierungsgruppc und Bildung von Massenspektrum-Fragmentionen, die dem Verlust von einem oder mehreren Kodierungsgruppen von der Kodierungssequenz entsprechen, zu bewirken, und danach Korrelieren der Peaks des Massenspektrums der Massenspektrum-Fragmentionen mit den Molekülionen des Fragments Y^{a} zum Identifizieren der Sequenz der einzelnen Kodierungsgruppen umfasst.

34. Verfahren nach Anspruch 33, wobei das Fragment F^{a} ein Mittel enthält, um dem Massenspektrum des Fragments eine charakteristische Signatur zu verleihen.

35. Verfahren zum Identifizieren eines pharmazeutisch geeigneten Substrats, umfassend das Erstellen einer Bibliothek, die eine Mehrzahl von chemischen Konstrukten nach einem der vorstehenden Ansprüche enthält, und biologisches Testen der Bibliothek, um die biologisch aktiven Substrate zu identifizieren.

36. Verfahren nach Anspruch 35, das den weiteren Schritt der Formulierung eines so identifizierten biologisch wirksamen Substrats mit einem pharmazeutisch verträglichen Träger unter Bilden eines Arzneimittels einschließt.

## Revendications

1. Assemblage chimique destiné à être utilisé dans une synthèse en phase solide, comprenant un support solide Q auquel sont liés des groupes Y¹R et Y²R ; dans lesquels R représente un substrat ou un marqueur de codage et les groupes Y¹ et Y² représentent des groupes de connexion ayant chacun un premier site de clivage, au moins un des groupes Y¹ et Y² ayant un second site de clivage situé entre le premier site de clivage et le groupe R, le premier site de clivage étant clivable de manière orthogonale et sélective par rapport au second site de clivage et, lorsque les deux groupes Y¹ et Y² contiennent un second site de clivage, le second site de clivage dans Y¹ étant clivable de manière sélective et orthogonale par rapport au second site de clivage dans Y² ; le second site de clivage étant clivable pour libérer le substrat ; et le premier site de clivage étant clivable sélectivement pour libérer un fragment Fr comprenant le substrat R et au moins une partie du groupe de connexion Y ; et dans lequel :
(i) le fragment chimique Fr contient un groupe sensibilisant G qui sensibilise le fragment chimique Fr à une analyse instrumentale, par exemple par spectroscopie de masse ; et/ou :
(ii) le fragment Fr contient un moyen pour conférer une signature caractéristique au spectre de masse du fragment.

2. Assemblage chimique suivant la revendication 1, dans lequel au moins un groupe R est un substrat.

3. Assemblage chimique suivant la revendication 1, comprenant un support solide Q auquel sont liés des groupes Y¹R et Y²R ; dans lesquels R représente un substrat (tel qu'une molécule médicamenteuse) et les groupes Y¹ et Y² représentent des groupes de connexion ayant chacun des premier et second sites de clivage qui sont clivables de manière orthogonale et sélective, le second site de clivage dans Y¹ étant clivable de manière sélective et orthogonale par rapport au second site de clivage dans Y² ; le second site de clivage étant clivable pour libérer le substrat ; et le premier site de clivage étant situé à une position entre le second site de clivage et le support solide et étant clivable sélectivement pour libérer un fragment Fr comprenant le substrat R et au moins une partie du groupe de connexion Y ; et dans lequel :
(i) le fragment chimique Fr contient un groupe sensibilisant G qui sensibilise le fragment chimique Fr à une analyse instrumentale, par exemple par spectroscopie de masse ; et/ou :
(ii) le fragment Fr contient un moyen pour conférer une signature caractéristique au spectre de masse du fragment.

4. Assemblage chimique suivant la revendication 1, répondant à la formule :

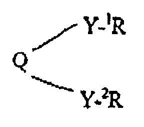

5. Assemblage chimique suivant l'une quelconque des revendications précédentes, dans lequel chaque support solide contient un marqueur de codage ou une séquence codante qui code pour l'information fournissant une indication sur au moins une partie de l'antécédent de synthèse de l'assemblage.

6. Assemblage chimique suivant la revendication 5, dans lequel le marqueur de codage est une séquence codante liée au support solide.

7. Assemblage chimique suivant la revendication 6, dans lequel la séquence codante est liée au support solide au moyen d'un groupe de connexion Y^{a} ayant un site de clivage clivable pour libérer un fragment F^{a} du support solide, le fragment F^{a} comprenant la séquence codante et facultativement au moins une partie du groupe de connexion Y^{a}.

8. Assemblage chimique suivant la revendication 7, dans lequel :
(i) le fragment chimique F^{a} contient un groupe sensibilisant G qui sensibilise le fragment chimique F^{a} à une analyse instrumentale, par exemple par spectroscopie de masse ; et/ou :
(ii) le fragment F^{a} contient un moyen pour conférer une signature caractéristique au spectre de masse du fragment.

9. Assemblage chimique suivant la revendication 7, dans lequel le site de clivage du groupe Y^{a} est clivable dans des conditions correspondant à celles requises pour cliver les premiers sites de clivage dans les groupes Y¹R et Y²R.

10. Assemblage chimique suivant la revendication 8, dans lequel le fragment chimique F^{a} contient un groupe sensibilisant G qui sensibilise le fragment chimique F^{a} à une analyse instrumentale, par exemple par spectroscopie de masse.

11. Assemblage chimique suivant l'une quelconque des revendications précédentes, dans lequel une proportion du substrat total R dans l'assemblage est liée au support solide au moyen d'un groupe de connexion Y^{b} ayant un site de clivage qui est clivable pour libérer un fragment F^{b} du support solide, le fragment F^{b} comprenant le substrat R et au moins une partie du groupe de connexion Y^{b} ; le groupe de connexion Y^{b} n'étant pas clivable pour libérer le substrat R dans des conditions efficaces pour cliver les seconds sites de clivage dans les groupes Y¹R et Y²R ; et dans lequel :
(i) le fragment chimique F^{b} contient un groupe sensibilisant G qui sensibilise le fragment chimique F^{b} à une analyse instrumentale, par exemple par spectroscopie de masse ; et/ou :
(ii) le fragment F^{b} contient un moyen pour conférer une signature caractéristique au spectre de masse du fragment.

12. Assemblage chimique suivant l'une quelconque des revendications précédentes, dans lequel le groupe sensibilisant G est engendré par clivage au niveau du premier site de clivage du groupe Y¹ ou Y² ou, lorsqu'il est présent, Y^{a} ou dudit site de clivage de Y^{b}.

13. Assemblage chimique suivant l'une quelconque des revendications précédentes, dans lequel le groupe sensibilisant G est un groupe amino basique ou un groupe carboxylate, de préférence un groupe amino basique.

14. Assemblage chimique suivant la revendication 13, dans lequel le groupe sensibilisant est un groupe amino primaire, un groupe amino secondaire ou un groupe amino tertiaire.

15. Assemblage chimique suivant la revendication 14, dans lequel le groupe sensibilisant est un groupe amino tertiaire choisi parmi des groupes amino cycliques tels que des groupes pipéridino, pipérazino (par exemple le N-méthylpipérazino), pyrrolidino ou morpholino, pipéridino.

16. Assemblage suivant l'une quelconque des revendications précédentes, dans lequel le fragment Fr et, (lorsqu'il est présent), facultativement le fragment F^{a} et (lorsqu'il est présent) facultativement le fragment F^{b} contiennent un moyen pour conférer une signature caractéristique au spectre de masse du fragment.

17. Assemblage suivant la revendication 16, dans lequel la signature est fournie en incorporant au fragment un marqueur isotopique "de scission de pic" comprenant au moins un atome qui existe sous un certain nombre de formes isotopiques stables.

18. Assemblage chimique suivant la revendication 17, dans lequel les fragments Fr et F^{a} et (lorsqu'il est présent) facultativement le fragment F^{b} sont marqués différemment de manière à produire des signatures caractéristiques différentes.

19. Assemblage chimique suivant la revendication 17 ou la revendication 18, dans lequel le marqueur isotopique comprend un ou plusieurs atomes choisis entre ¹H/H² (D), ⁷⁹Br/⁸¹Br, ¹²C/¹³C, ¹⁴N/¹⁵N et ¹⁶O/¹⁸O.

20. Assemblage chimique suivant l'une quelconque des revendications 17 à 19, dans lequel le ou les marqueurs isotopiques sont situés entre les premier et second sites de clivage des groupes Y¹ et Y².

21. Assemblage chimique répondant à la définition suivant l'une quelconque des revendications précédentes, dans lequel les premier et second sites de clivage dans les groupes Y¹ sont définis par des premier et second groupes de liaison L¹ et L², les premier et second sites de clivage dans le groupe Y² sont définis par des premier et second groupes de liaison L¹ et L³, le site de clivage dans le groupe Y^{a} (lorsqu'il est présent) est défini par un groupe de liaison L^{a} et le site de clivage dans le groupe Y^{b} (lorsqu'il est présent) est défini par un groupe de liaison L^{b}.

22. Assemblage chimique suivant la revendication 21, dans lequel les groupes de liaison L^{a} et L^{b} correspondent à L¹.

23. Assemblage chimique suivant la revendication 21 ou la revendication 22, dans lequel un groupe intercalaire A est interposé entre les différentes paires des premier et second groupes de liaison, ou entre le groupe de liaison L^{a} et le marqueur de codage, ou entre le groupe de liaison L^{b} et le substrat R, le groupe intercalaire A contenant un marqueur de scission de pic isotopique.

24. Assemblage chimique suivant l'une quelconque des revendications précédentes, répondant à une formule choisie dans le groupe consistant en les formules suivantes : 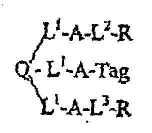 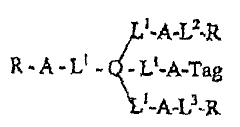 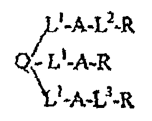 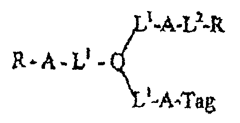
Tag-A-L¹-Q-L¹-A-L²-R
R-A-L¹-Q-L¹-A-L²-R 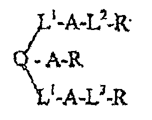 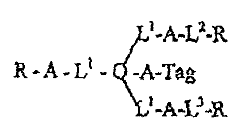 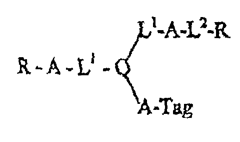 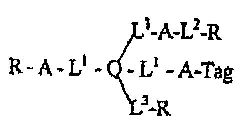 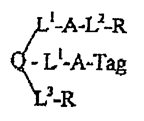 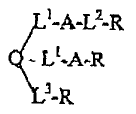
R-L³-Q-L¹-A-L²-R 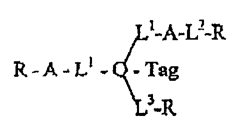
dans lesquelles L¹, L², L³, A et R répondent aux définitions dans l'une quelconque des revendications précédentes et le terme "Marqueur" représente une séquence codante.

25. Assemblage suivant l'une quelconque des revendications 1 à 24, destiné à être utilisé dans une méthode de sélection à libération par paliers, l'assemblage répondant à la formule Marqueur - A - L¹ - Q - L¹ - A - L² - R dans laquelle le terme Marqueur, A, L¹, Q, L² et R répondent aux définitions dans l'une quelconque des revendications précédentes.

26. Assemblage chimique suivant l'une quelconque des revendications précédentes, dans lequel les sites de clivage, clivables de manière orthogonale, peuvent être clivés par une réaction choisie entre un clivage catalysé avec un acide, un clivage catalysé avec une base, un clivage oxydatif, un clivage réducteur, un déplacement nucléophile, un déplacement électrophile et un clivage thermique, photochimique ou enzymatique.

27. Assemblages chimiques intermédiaires destinés à être utilisés dans la préparation d'un assemblage chimique répondant à la définition dans l'une quelconque des revendications précédentes, les assemblages intermédiaires répondant aux formules Y¹-Q-Y², RY¹-Q-Y² et Y¹-Q-Y²R dans lesquelles Y¹ et Y² représentent des formes réactives ou protégées du groupe Y ; et R, Q et Y répondent aux définitions dans l'une quelconque des revendications précédentes.

28. Assemblages intermédiaires de formules L²-A-L¹-Q-L¹-A^{p}, R-L²-A-L¹-Q-L¹-A^{p}, L^{3'}-A-L¹-Q-L¹-A^{p}, R-L³-A-L¹-Q-L¹-A^{p}, R-L³-A-L¹-Q-L¹-A-L² et L³-A-L¹-Q-L¹-A-L²-R dans lesquelles L¹, L² et L³ représentent des formes réactives ou protégées des groupes de liaison L¹, L² et L³, A^{p} représente une forme réactive ou protégée du groupe intercalaire A contenant un marqueur isotopique de scission de pic et Q, R, A, L¹, L² et L³ répondent aux définitions dans l'une quelconque des revendications précédentes.

29. Assemblage intermédiaire suivant la revendication 28, dans lequel le groupe A^{p} répond à la formule NH-Alk-NX¹ dans laquelle Alk représente un groupe alkylène et X¹ représente l'hydrogène ou un groupe aralkyle.

30. Assemblage intermédiaire suivant la revendication 28 ou la revendication 29, dans lequel le support solide porte, à l'état lié à celui-ci, une séquence de marqueur de codage L¹-A-Marqueur et/ou une séquence R-A-L¹-, ou une forme de précurseur de celle-ci.

31. Méthode d'analyse, à libération différentielle, une banque chimique pour la détermination d'une activité biologique, méthode comprenant les étapes consistant :
(i) à soumettre un assemblage comprenant un support solide Q auquel sont liés des groupes Y¹R et Y²R répondant aux définitions suivant l'une quelconque des revendications précédentes à des conditions de clivage efficaces pour libérer le substrat R du groupe Y¹R ;
(ii) à tester le substrat R libéré du groupe Y¹R dans un essai biologique ;
(iii) à soumettre ensuite l'assemblage à des conditions de clivage efficaces pour libérer le substrat R du groupe Y²R ; et
(iv) à tester le substrat R libéré du groupe Y²R dans un essai biologique.

32. Méthode d'analyse, par libération par paliers, d'une banque chimique pour la détermination d'une activité biologique, méthode comprenant les étapes consistant :
(i) à soumettre un assemblage suivant l'une quelconque des revendications 1 à 26 à des conditions de clivage efficaces pour libérer une première portion du substrat R du groupe Y¹R ;
(ii) à tester la première portion du substrat R libérée du groupe Y¹R dans un essai biologique ;
(iii) à soumettre l'assemblage à des conditions de clivage efficaces pour libérer une seconde portion du substrat R du groupe Y²R ; et
(iv) à tester la seconde portion de substrat R libérée du groupe Y²R dans un essai biologique.

33. Méthode de détermination de l'identité d'un substrat R lié à un support solide Q d'un assemblage suivant l'une quelconque des revendications 7 à 26 par un moyen de spectrométrie de masse ; le support solide Q comportant une séquence codante fixée à celui-ci au moyen d'un groupe de connexion Y^{a} ayant un site de clivage clivable pour libérer un fragment F^{a} du support solide, le fragment F^{a} comprenant la séquence codante et au moins une partie du groupe de connexion Y^{a}, dans laquelle (i) le fragment chimique F^{a} contient un groupe sensibilisant G qui sensibilise le fragment chimique F^{a} à une analyse par spectroscopie de masse ;
la séquence codante comprenant une séquence de groupes codants dont la nature et l'ordre fournissent une indication de l'identité du substrat R ;
méthode comprenant les étapes consistant à cliver le groupe de connexion Y^{a} de manière à libérer le fragment F^{a} du support solide ; à soumettre le fragment Y^{a} à une analyse par spectrométrie de masse dans des conditions efficaces pour provoquer la fragmentation de spectrométrie de masse du groupe codant et la formation d'ions de fragments de spectrométrie de masse correspondant à la perte d'un ou plusieurs groupes codants par la séquence codante, et ensuite à corréler les pics de spectrométrie de masse des ions de fragments de spectrométrie de masse avec l'ion moléculaire du fragment Y^{a} pour identifier la séquence des différents groupes codants.

34. Méthode suivant la revendication 33, dans laquelle le fragment F^{a} contient un moyen pour conférer une signature caractéristique au spectre de masse du fragment.

35. Méthode pour identifier un substrat pharmaceutiquement utile, comprenant les étapes consistant à préparer une banque contenant une pluralité d'assemblages chimiques répondant à la définition suivant l'une quelconque des revendications précédentes, et à soumettre la banque à un test biologique pour identifier les substrats biologiquement actifs.

36. Méthode suivant la revendication 35, qui comprend l'étape supplémentaire consistant à formuler un substrat biologiquement actif ainsi identifié avec un support pharmaceutiquement acceptable pour produire une composition pharmaceutique.
